(19) Europäisches Patentamt
European Patent Office
Office européen des brevets

(11) **EP 3 909 209 B1**

(12) **EUROPEAN PATENT SPECIFICATION**

(45) Date of publication and mention of the grant of the patent:
**31.05.2023 Bulletin 2023/22**

(21) Application number: **20728369.8**

(22) Date of filing: **09.05.2020**

(51) International Patent Classification (IPC):
**H04L 25/49** *(2006.01)*

(52) Cooperative Patent Classification (CPC):
**H04L 25/4908**

(86) International application number:
**PCT/US2020/032263**

(87) International publication number:
**WO 2020/231877 (19.11.2020 Gazette 2020/47)**

(54) **ETHERNET SIGNAL FORMAT USING 256B/257B BLOCK ENCODING**

ETHERNET SIGNALFORMAT MIT 256B/257B BLOCKCODIERUNG

FORMAT DE SIGNAL ETHERNET UTILISANT UN CODAGE DE BLOCS DE TYPE 256B/257B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2019 US 201916408576**

(43) Date of publication of application:
**17.11.2021 Bulletin 2021/46**

(73) Proprietor: **Ciena Corporation**
**Hanover, MD 21076 (US)**

(72) Inventors:
• **GAREAU, Sebastien**
**Ottawa, ON K2K 0L1 (CA)**
• **MANILOFF, Eric**
**Ottawa, ON K2K 0L1 (CA)**

(74) Representative: **Dehns**
**St. Bride's House**
**10 Salisbury Square**
**London EC4Y 8JD (GB)**

(56) References cited:
**US-A1- 2016 182 084**

• **JEAN-MICHEL CAIA FIBERHOME P R CHINA: "FlexO25n Multiplexing and Overhead structures for 25G/50G M-OTN interfaces;WD11-24", ITU-T DRAFT; STUDY PERIOD 2017-2020; STUDY GROUP 15; SERIES WD11-24, INTERNATIONAL TELECOMMUNICATION UNION, GENEVA ; CH , vol. 11/15,12/15 28 May 2018 (2018-05-28), pages 1-16, XP044246742, Retrieved from the Internet: URL:https://www.itu.int/ifa/ifa/t/2017/sg1 5/exchange/wp3/q11/2018-06-Beijing/WD11-24 -FiberHome-CT-25G_50G_FlexO25n_Mux_and_ OH_ Structures.docx [retrieved on 2018-05-28]**
• **Anonymous: "Flex Ethernet Implementation Agreement", OIF , OPTICAL INTERNETWORKING FORUM, 21 June 2017 (2017-06-21), pages 1-35, XP055459219, Retrieved from the Internet: URL:http://www.oiforum.com/wp-content/uplo ads/FLEXE1.1.pdf [retrieved on 2018-03-14]**

**Description**

**FIELD OF THE DISCLOSURE**

The present disclosure generally relates to Ethernet networking. More particularly, the present disclosure relates to systems and methods for an Ethernet frame format using 256b/257b block encoding.

**BACKGROUND OF THE DISCLOSURE**

FlexE (Flex Ethernet) is currently defined in the Optical Internetworking Forum (OIF) such as via the Flex Ethernet 2.0 Implementation Agreement, IA #OIF-FLEXE-02.0, June 22, 2018, the Flex Ethernet 1.1 Implementation Agreement, IA #OIF-FLEXE-01.1, June 21, 2017, and the Flex Ethernet 1.0 Implementation Agreement, IA #OIF-FLEXE-01.0, March 2016. FlexE allows for flexible Ethernet connectivity between routers and optical transport equipment that is independent of the physical interfaces between the two devices. The net benefit is the ability to remotely adjust service bandwidth as needed, more efficiently utilize each networking layer, and improve end-to-end manageability of the network. FlexE dissociates the Ethernet rate on the client side from the actual physical interface (also called server) by introducing a new shim through the IEEE defined Media Access Control (MAC) and Physical Coding Sublayer (PCS) layers. The current FlexE standard is defined for 100G PHYs, with 200G/400G PHYs being defined in subsequent Implementation Agreements.

MTN (metro transport networking, G.mtn) is being developed in the International Telecommunication Union (ITU) as G.mtn "Interfaces for a metro transport network." MTN has also been historically called "Flexible Ethernet (FlexE) switching" or Sliced packet Networking (SPN). MTN is a layer 1 transport technology similar to Optical Transport Network (OTN). MTN is being developed for 5G transport, and MTN fundamentally utilizes Ethernet and 64b/66b blocks. 64b/66b is a line code that transforms 64-bit data to a 66-bit line code to provide enough state changes to allow reasonable clock recovery and alignment of the data stream at the receiver. 64b/66b was defined by the IEEE 802.3 working group as part of the IEEE 802.3ae-2002 amendment which introduced 10 Gbit/s Ethernet. An output of 64b/66b encoding is a 66b block. The driving application for MTN is 5G mobile transport, and there is a need for timing support in such MTN networks, utilizing the 66b blocks.

64b/66b is a line code that transforms 64-bit data to 66-bit line code to provide enough state changes to allow reasonable clock recovery and alignment of the data stream at the receiver. It was defined by the IEEE 802.3 working group as part of the IEEE 802.3ae-2002 amendment which introduced 10 Gbit/s Ethernet. The overhead of 64b/66b encoding is 2 coding bits for every 64 payload bits or 3.125%. FlexE has a frame structure (Time Division Multiplexing (TDM) structure) based on 66b block boundaries in 64b/66b encoding. FlexE is designed to work with legacy (no Forward Error Correction (FEC)) 100G PCS/PHY that are based on 64b/66b encoding.

Ethernet blocks, such as FlexE, MTN, etc., encoded with 64b/66b encoding is wasteful in bandwidth compared to 256b/257b encoding which is being used in newer standards such as for 400 GbE (Gigabit Ethernet) including OIF 400ZR. A 256b/257b encoding provides a ~3% improvement in bandwidth relative to 64b/66b encoding. Current FlexE with Optical Transport Network (OTN) mapping procedures are all based on 66b since they need access to the individual FlexE overhead blocks and calendar slots. Standard transcoding FlexE 66b blocks into a 257b PCS results in a non-optimal structure. The FlexE overhead will float inside a 257b block and will change position (1 out of 4). It is not obvious to locate the FlexE OH and frame boundaries without having to trans-decode back to 66b. The FlexE clients or calendar slots are distributed on 66b boundaries and mixed within 257b blocks.

For designs that require mapping Ethernet signals to OTN (or OIF 400ZR) and want to keep data signals in the 257b transcoded format to save on bandwidth, the complexities mentioned above will lead to latency, extra complexity, logic, and power. OIF 400ZR has been defined to transport transcoded Ethernet data directly to save on bandwidth, and will likely result in a class of ZR type of solutions that optimize power and spectral utilization by directly transporting transcoded Ethernet. While these solutions can be used to transport FlexE, they do not provide a means of rate-reduction by dropping the unused timeslots.

Jean-Michel Caia Fiberhome PR China describes "Flex025n Multiplexing and Overhead structures for 25G/50G M-OTN interfaces; WD11-24", ITU-T draft; vol. 11 /15, 12/15 28 May 2018, pages 1-16, XP0442467 42, retrieved from the Internet: URL:https://www.itu.int/ifa/ifa/t/2017/sg15/exchange/wp3/q11 /2018-06-Beijing/WD11-24-FiberHome-CT-25G_50G_Flex025n_Mux'-and_OH_Structures.docx [retrieved on 2018/05/28]

**BRIEF DESCRIPTION OF THE DRAWINGS**

The present disclosure is illustrated and described herein with reference to the various drawings, in which like reference numbers are used to denote like system components/method steps, as appropriate, and in which:

FIG. 1 is a block diagram of a FlexE frame currently defined in the Optical Internetworking Forum (OIF) illustrating insertion of FlexE overhead on each 100G FlexE Instance of a FlexE Group;
FIG. 2 is a block diagram of a FlexE overhead frame and multiframe;
FIG. 3 is a block diagram of a 257b block that is a Data Block (DB);
FIG. 4 is a block diagram of a 257b block that is a Control Block (CB);
FIG. 5 is a block diagram of a new FlexE frame based on 257b blocks;
FIG. 6 is a block diagram of FlexE frames with the 257b overhead block and the 257b data blocks carrying the same data as the FlexE frame that uses 66b blocks;
FIG. 7 is a block diagram of four 257b blocks for the FlexE 50 representing eight x 66b blocks for a multiframe from the FlexE frame;
FIG. 8 is a block diagram of a mapping circuit and a demapping circuit for interfacing the FlexE frame at 66b blocks with the FlexE frame at 257b blocks;
FIG. 9 is a block diagram of mapping four Data Blocks (DB) at 66b into one 257b block;
FIG. 10 is a block diagram of mapping four Control Blocks (CB) at 66b into one 257b block;
FIG. 11 is a flowchart of a process for mapping the FlexE frame with 66b blocks into the FlexE frame with 257b blocks;
FIG. 12 is a flowchart of a process for mapping the FlexE frame with 257b blocks;
FIGS. 13 and 14 are block diagrams of an Ethernet frame switch showing FlexE transparent (FIG. 13) and showing FlexE termination (FIG. 14);
FIG. 15 is a block diagram of processing performed by the line cards of the switch of FIGS. 13 and 14;
FIG. 16 is a block diagram of an STS switch adapted to support Ethernet frames;
FIGS. 17 and 18 are block diagrams illustrate an OFP switch adapted to support Ethernet frame switching;
FIG. 19 is a network diagram illustrates a comparison of FlexO service CAM with the FlexE Client service CAM systems and methods described herein;
FIG. 20 is a network diagram of a network with various client CAM monitoring points;
FIG. 21 is a flowchart of a process, implemented in a node, for monitoring a client service that uses a 257b block-based frame in a network;
FIG. 22 is a diagram of a differential timing process between two nodes utilizing FlexE;
FIG. 23 is a flowchart of a process, implemented in a node, for supporting support multi-service with Flexible Ethernet (FlexE), MTN, etc.;
FIG. 24 is a logical diagram of a protocol stack for Layers 1-3;
FIG. 25 is a logical diagram of use of FlexE in a C2C application;
FIG. 26 is a flowchart of a time transfer process using a stream of Ethernet blocks;
FIG. 27 is a network diagram of a network with node A and node B connected to one another via a stream of Ethernet blocks;
FIG. 28 is a flowchart of a time transfer method between a first node and a second node;
FIG. 29 is a network diagram of a terminating transport network with network elements coupled to switches/routers and with intermediate, transparent network elements between the network elements, with encryption on a stream of 257b blocks;
FIG. 30 is a table of 256b/257b block formats; and
FIG. 31 is a flowchart of a process for Physical Coding Sublayer (PCS) encryption implemented by a first network element communicatively coupled to a second network element.

## DETAILED DESCRIPTION OF THE DISCLOSURE

In various embodiments, the present disclosure relates to systems and methods for an Ethernet frame (signal) format using 256b/257b block encoding. Specifically, the systems and methods can reorder the existing FlexE frame structure based on 64b/66b encoding into something that is convenient for 257b PHYs and mappers. Advantageously, the systems and methods described herein provide efficient bandwidth mapping, sub-rating, overhead access and FlexE awareness in the 256b/257b block encoding, support for 4x100GbE in a 400ZR, and legacy 64b/66b interoperability. For the efficient bandwidth mapping, there is momentum in the industry to keep Ethernet signals in their transcoded format to save on bandwidth. For example, OIF 400ZR keeps 400GbE in its 257b format when mapping to the ZR frame. A new design interfacing directly to a gray 400GE PHY or a 400ZR optical module could skip the 64b/66b encoding step altogether and create a FlexE signal in the 257b native form altogether, saving power, latency, and circuitry. Note, the present disclosure is described with reference, for example, to FlexE and a FlexE frame format, but those skilled in the art will recognize the systems and methods described herein contemplate use with any protocol utilizing a stream of blocks to transport Ethernet, including, G.mtn or the like. That is, the systems and methods described herein can be used with any Ethernet frame format that utilizes 256b/257b encoding.

For sub-rating, there is a movement to add Ethernet mappings in the 257b format instead of always reverting to 66b as is the current standard. The "crunching" or sub-rating would be greatly simplified since all the unequipped calendar slots are now grouped into common 257b blocks. The circuitry can simply drop 257b blocks that are not mapped into an Optical Data Unit flex (ODUflex). Again, it removes the need for trans-decoding back to 66b, saves on power, latency, and circuitry. In applications that need access to FlexE overhead, for example, neighbor discovery or Precision Time Protocol (PTP), the FlexE overhead 257b block can easily be located. For 400ZR modules, adding FlexE awareness can be greatly simplified, and would not require trans-decoding back to 66b. Again, savings include power, latency, and circuitry.

There is a push to support 4x100GbE clients in 400ZR applications. The FlexE frame format could be used as a multiplexing service layer where four clients are mapped directly into grouped 5x257b calendar slots, and this structure is then be adapted to the 400ZR frame format, saving power, latency, and circuitry. Finally, for legacy 66b interoperability, new designs can optimize and implement the 257b FlexE systems and methods described herein, and the burden of the legacy 66b support falls to interworking scenarios with 100GbE (no FEC) interfaces. 257b is aligned to common bus width found in the latest 16/7nm devices.

All new (beyond 100G) Ethernet PHYs require FEC and are based on 256b/257b encoding. Additionally, 100GbE Ethernet has been extended with 257b for newer standards. Many new designs do not actually implement 64b/66b encoding anymore and directly adapt a packet stream (via Medium Independent Interface (MII)) into 257b PCS encoding. As mentioned above, the 66b FlexE frame 10 can be transcoded easily into 257b. However, there are drawbacks as described herein, namely latency, extra complexity, circuitry, and power.

Additionally, the present disclosure includes switching implementation for 257b blocks; Operations, Administration, and Maintenance (OAM) techniques for 257b blocks; multi-service and timing transparency for 257b blocks; Chip-to-Chip (C2C) interface systems and methods for 257b blocks; time transfer techniques for 257b blocks; and encryption for 257b blocks.

### FlexE frame based on 66b

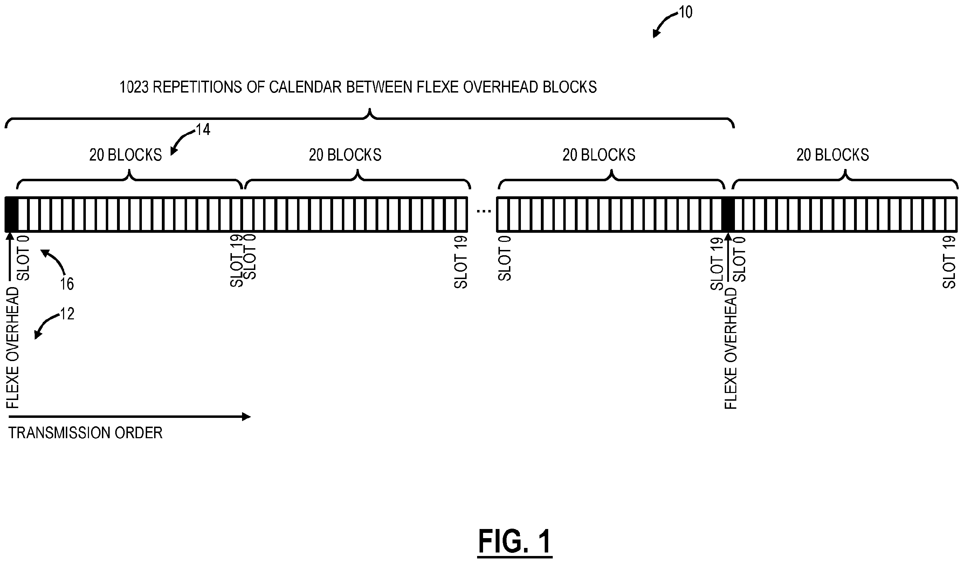

FIG. 1 is a block diagram of a FlexE frame 10 currently defined in the OIF illustrating insertion of FlexE overhead 12 on each 100G FlexE Instance of a FlexE Group. In the FlexE frame 10, a frame/overhead block 12 (which is a 66b block) is located every 20*1023 blocks 14 (which are also 66b blocks). After the frame/overhead block 12, the frame payload is also then divided into 20 66b calendar slots 16. Note, the blocks 14 are the data in the calendar slots 16.

The FlexE mechanism operates using a calendar which assigns 66B block 14 positions on sub-calendars on each 100G FlexE Instance of the FlexE Group to each of the FlexE Clients. The calendar is described with a granularity of 5G (although implementations may have a different granularity, e.g., 25G), and has a length of twenty 5G slots 16 per 100G of FlexE Group capacity. Two calendar configurations are supported: an "A" and a "B" calendar configuration. At any given time, one of the calendar configurations is used for mapping the FlexE Clients into the FlexE Group and demapping the FlexE Clients from the FlexE Group. The two calendar configurations are provided to facilitate reconfiguration. When a switch of calendar configurations adds orremoves FlexE Clients from the FlexE Group, existing clients whose size and calendar slot 16 assignments are not changed by changing the calendar configuration are not affected.

For a FlexE Group composed of n 100G FlexE Instances, the logical length of the calendar is 20n. The blocks 14 as allocated per the calendar are distributed to n sub-calendars of length 20 on each of the 100G FlexE Instances of the FlexE Group. The order of distribution of twenty blocks 14 at a time is selected over simple "round robin" distribution of 66B blocks 14. Calendar slots 16 are identified by their 100G FlexE Instance number and the slot [0-19] (within that 100G FlexE Instance). The "logical" sequence number of a calendar slot 16 is $20\times$ the instance number plus the calendar slot number within the 100G FlexE Instance. The sequence is in ascending order. Note that the sequence numbering is not necessarily consecutive when the assigned PHY numbers are not contiguous. This logical order only matters when calendar slots on different 100G FlexE Instances are assigned to the same FlexE Client.

The alignment of the data from the 100G FlexE Instances of the FlexE Group is accomplished by the insertion of FlexE overhead 12 into the stream of 66B blocks 14 carried over the group. The FlexE overhead 12 is delineated by a 66B block which can be recognized independently of the FlexE Client data. An illustration of the FlexE overhead on each 100G FlexE Instance of the FlexE Group is given in FIG. 1.

On a 100G FlexE Instance, the FlexE overhead 12 block will occur approximately once per 13.1$\mu$s. The actual format of the FlexE overhead 12 blocks is such that they occur in a repeating sequence of eight blocks, so the sequence has a period of approximately 104.77$\mu$s. This sequence of overhead blocks is inserted in the same positions in the sub-calendar sequence on each 100G FlexE Instance and is used to align all of the 100G FlexE Instances of the FlexE Group at the FlexE demux to reconstruct the sequence in the order of the calendar so that the FlexE Clients can be recovered.

The anchor position of the FlexE overhead 12 on each 100G FlexE Instance is encoded as an ordered set (control block type 0x4B). A distinct "O" (operational) code is used (0x5) which is different from that for the sequence ordered set used by Ethernet or the signal ordered set used by Fibre channel. The information to be transmitted in the FlexE overhead is encoded into the bytes D1, D2, and D3 of the ordered set block.

The information which needs to be included in the overhead 12 includes:

Which 100G FlexE Instances are part of this FlexE Group;

The number identifying this 100G FlexE Instance within the FlexE Group. Note that multiple 100G FlexE Instances carried on a 200GBASE-R or 400GBASE-R each have a different 100G FlexE Instance number constructed using the PHY number, while for 100GBASE-R PHYs, the 100G FlexE Instance number and the PHY number are the same. (100G FlexE Instance numbers are unique within a FlexE Group. They are independent between FlexE Groups. Two 100G FlexE instances in different FlexE Groups may have the same 100G FlexE Instance number.);

A way to transmit the programming of the sub-calendar configurations for each 100G FlexE Instance from the FlexE mux to the FlexE demux;

A way to indicate which calendar configurations ("A" or "B") is in use at this time;

The FlexE Group number that this 100G FlexE instance is a member of (if necessary, for the case where the same 100G FlexE Instance number may be configured to be part of different FlexE Groups);

Fields to support protocols necessary for changing the configuration of FlexE Clients into calendar slots 16;

Two optional management channels. These may not be necessary in all applications (for example, if a network management system has direct access to the FlexE Shim at both ends of the connection), but may be useful for applications such as using FlexE for an n × 100G umbilicus to a remote shelf of lower-rate ports. One management channel (the 4th and 5th blocks of the FlexE overhead 12 frame of the first 100G FlexE Instance on a given PHY) is available for communication across a section (for example, from a router with a FlexE Shim to FlexE aware transport equipment which does not terminate the FlexE Shim), and the other management channel (the 6th-8th or 7th-8th blocks of the FlexE overhead frame of the first 100G FlexE Instance on a given PHY) is used for communication between the FlexE Shims; and

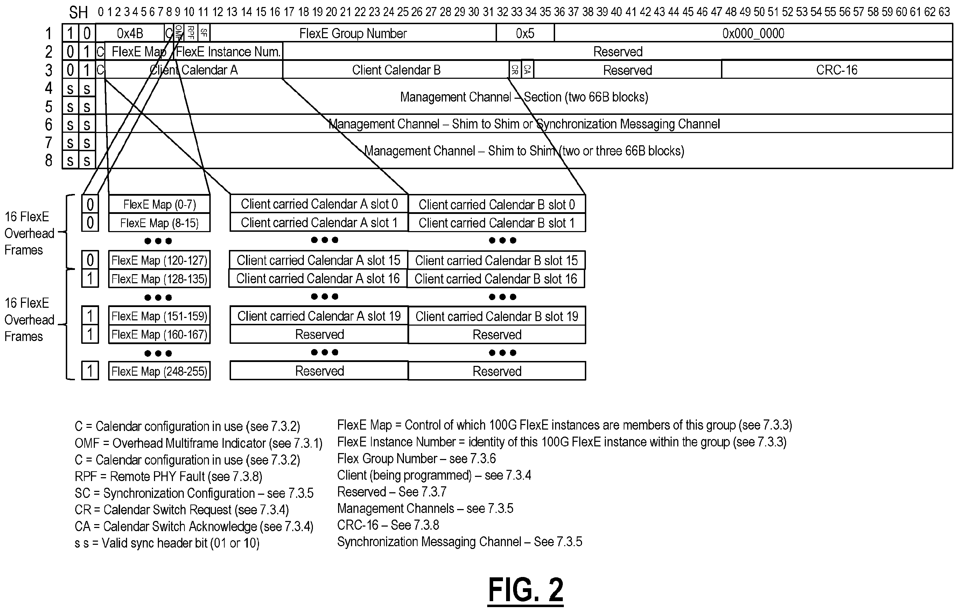

An optional synchronization messaging channel. If configured, this is carried in the 6th block of the FlexE overhead frame of the first 100G FlexE Instance on a given PHY. The amount of information to be conveyed from the FlexE mux to the FlexE demux exceeds the 24 bits available in a single ordered set block per 100G FlexE Instance. This is addressed by spreading the relevant overhead across a sequence of eight FlexE overhead blocks on each 100G FlexE Instance, each separated by 20 × 1023 FlexE data blocks. This group of eight overhead blocks is referred to as the FlexE overhead 12 frame. The FlexE overhead frame and multiframe is illustrated in FIG. 2. The first block 14 of the FlexE overhead 12 frame is encoded as an ordered set. The next two FlexE overhead 12 blocks 14 are encoded as data 66B data blocks. The final five FlexE overhead blocks 14 are reserved for the two optional management channels, and may carry any legal 66B block per [802.3] Figure 82-5 (excluding Ordered Sets with O code=0x5, as this is reserved for 100G FlexE Instance overhead).

The first block in the FlexE overhead 12 frame serves as a marker to be used for alignment and re-interleaving of the sub-calendars from each of the 100G FlexE Instances of the FlexE Group at the FlexE demux. One FlexE overhead frame consisting of eight 66B overhead blocks is transmitted in approximately 104.77 μs. Subject to the amount of buffer provided in a given implementation, skew detection and compensation across the 100G FlexE Instances of the FlexE Group can be compensated up to nearly half of this amount.

In the FlexE frame 10, the FlexE overhead 12 is encoded as 66B blocks and are inserted on each 100G FlexE Instance of the FlexE Group. One overhead 12 block is inserted after every 1023 iterations of the length 20 sub-calendar of 66B FlexE data blocks 14, so the sequence is one block of overhead 12 followed by 1023 × 20 blocks of data followed by one block of overhead 12.

FlexE overhead frame lock is achieved at the receiver (FlexE demux) on each PHY by recognizing the FlexE block 1 of the FlexE overhead frame, encoded as a special ordered set (the sync header is 10, the control block type is 0x4B (ordered set), and the "O" code is 0x5), and then finding the FlexE ordered set block again (1023 × 20 + 1) × 8 block positions later. Once FlexE overhead frame lock is achieved, the next expected FlexE overhead block will be 1023 × 20 + 1 block positions later. While in FlexE overhead frame lock, bytes D1-D3 of the ordered set block, plus the 66B blocks occurring at 20461, 40922, 61383, 81844, 102305, 122766, and 143227 blocks beyond the ordered set block will be interpreted as FlexE overhead frame. FlexE overhead is not interpreted if not in FlexE overhead lock. FlexE overhead lock will be lost if the sync header, control block type, or O code do not match at the expected position for 5 occurrences.

Certain information is transmitted in every FlexE overhead frame. Other information is distributed across a sequence of 32 FlexE overhead frames, referred to as the FlexE overhead multiframe. The OMF (overhead multiframe) bit has a value of "0" for the first sixteen overhead frames of the overhead multiframe, and a value of "1" for the last sixteen overhead frames of the overhead multiframe. The FlexE demux achieves overhead multiframe lock on each 100G FlexE Instance when the OMF bit transitions from a "0" to a "1" or a "1" to a "0" in consecutive overhead frames with good CRC. There are two opportunities in the overhead multiframe for the FlexE demux to achieve overhead multiframe lock.

**New FlexE frame format based on 257b**

The present disclosure proposes a new FlexE frame format with 257b block sizes and optimized for this new encoding, but compatible with the 66b encoding of the FlexE frame 10. This new FlexE frame format skips the intermediate step of 66b encoding and can be adapted directly on new Ethernet PCS/PHYs. The legacy FlexE frame 10 can be directly adapted to the new 257b FlexE frame format, and this shuffling is explained later.

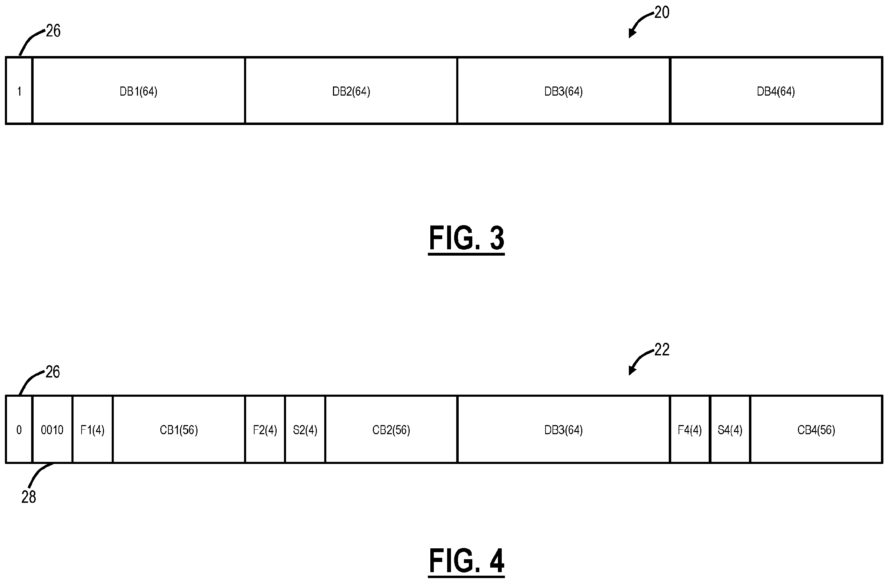

FIG. 3 is a block diagram of a 257b block 20 that is a Data Block (DB). FIG. 4 is a block diagram of a 257b block 22 that is a Control Block (CB). The 257b block as defined in IEEE 802.3 is as follows: a header bit 26 indicates if the block 20, 22 contains control blocks (CB) or only data blocks (DB). If the header bit 26 is 1, then the remaining 256 bits are data, e.g., four DB1 - DB4 as illustrated in FIG. 3. If the header bit 26 is 0, then the next 4-bit field 28 provides a mask for which of the four 64-bit words are CB versus DB. For example, FIG. 4 has a mask of 0010 meaning the first two blocks are CB, the third block (DB3) is a DB, and the fourth block is CB.

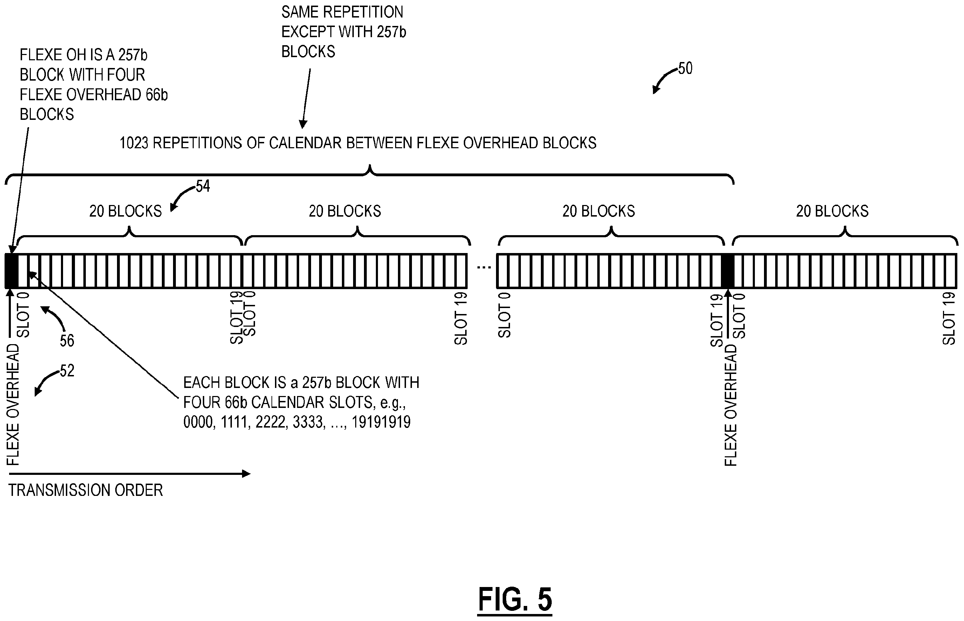

FIG. 5 is a block diagram of a new FlexE frame 50 based on 257b blocks 54. In an embodiment, the new FlexE frame 50 natively supports 256b/257b encoding and uses a similar structure as the existing FlexE frame 10 with 66b blocks. Specifically, the FlexE frame 50 includes overhead 52 blocks that are 257b followed by 20*1023 data blocks 54 that are also 257b and include slots 56 0 to 19. This is possible as the 257b blocks have 256b of data or control and the 66b blocks have 64b of data or control, i.e., the 256b of data and control is a multiple of 64b.

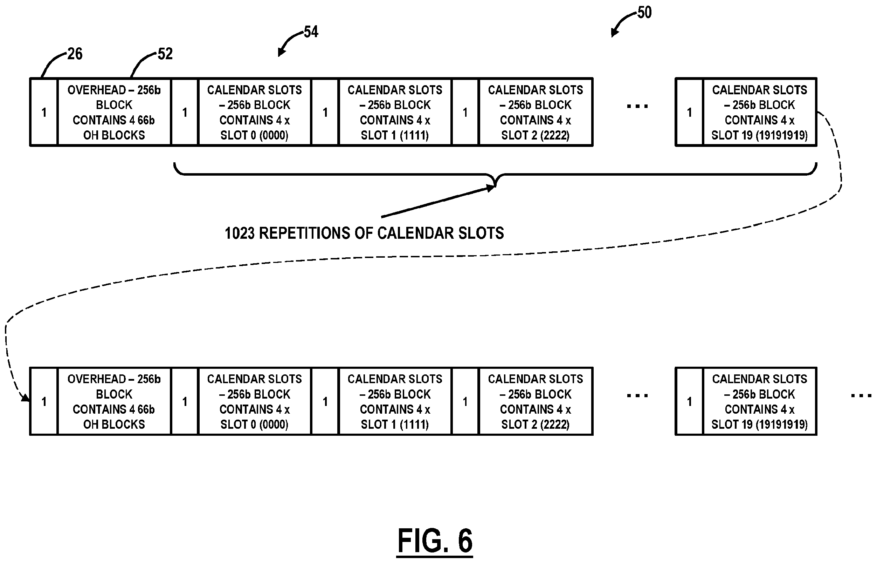

In another embodiment, the new FlexE frame 50 includes a shuffling or reordering of the FlexE frame 10 into something that is efficient for 257b PHYs and mappers. FIG. 6 is a block diagram of FlexE frames 50 with the 257b overhead block 52, and the 257b data blocks 54 carrying the same data as the FlexE frame 10 that uses 66b blocks. The new FlexE frame 50 would use an entire 257b block 52, 54 and essentially defines 4 × CBs (or DBs), each of these resembling the format already defined in the FlexE frame 10.

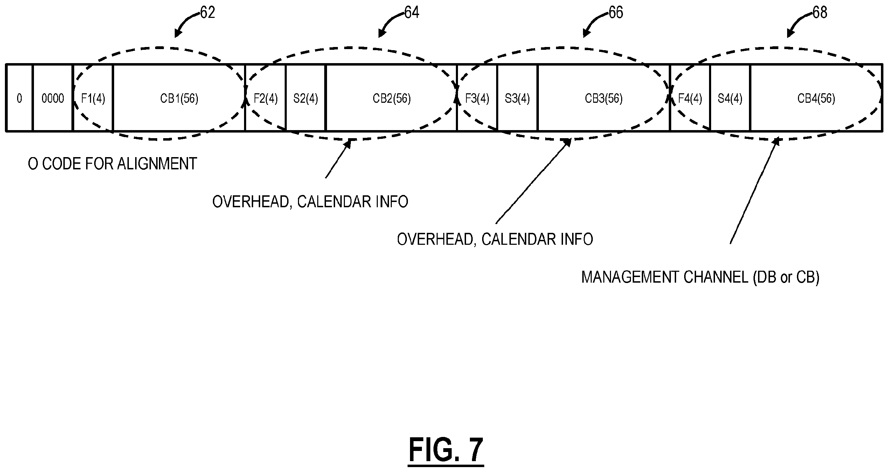

For the FlexE overhead 52, this can include four of the FlexE overhead 12 66b blocks concatenated into a single 257b block for the FlexE frame 50. Also, since the current FlexE standard based on 66b blocks define eight × 66b blocks for a multiframe, the new FlexE frame 50 can utilize two × 257b blocks for the multiframe (see FIG. 2). FIG. 7 is a block diagram of four 257b blocks 62, 64, 66, 68 for the FlexE frame 50 representing eight × 66b blocks for a multiframe from the FlexE frame 10. Here, the new FlexE frame 50 concatenates eight × 66b blocks from the multiframe from the FlexE frame 10 into two × 257b blocks.

Again, the multiframe from the FlexE frame 10 includes eight 66b blocks (see FIG. 2). The first block 14 of the FlexE overhead 12 frame is encoded as an ordered set. The next two FlexE overhead 12 blocks 14 are encoded as data 66B data blocks. The final five FlexE overhead blocks 14 are reserved for the two optional management channels, and may carry any legal 66B block per [802.3] Figure 82-5 (excluding Ordered Sets with O code=0x5, as this is reserved for 100G FlexE Instance overhead).

FIG. 7 illustrates the first four 257b blocks in the multiframe from the FlexE frame 10. The 257b block 62 includes four of the first block from FIG. 2 which is an O-code for alignment. The 257b blocks 64, 66 include four of the second and third bocks from FIG. 2, respectively, which include overhead and calendar information. The 257b block 68 includes four of the fourth block from FIG. 2 which is a management channel. The 257b block 68 can be a DB or CB. Not shown is the second of the two x 257b blocks, and this includes four of the fifth through eight blocks from FIG. 2 which are management channels.

In FIG. 6, the occurrence between the frame/OH blocks 52, 54 is still 20*1023 blocks, although, in this case, 257b blocks are used since each OH block carries 4 of the previous OH blocks. The framing algorithm should provide similar behavior since the occurrence of the O-code (within the 257b block) does not change. The payload 257b blocks can be composed with a mix of CB and DB depending on the FlexE client data. The calendar can be grouped and be the same # within the 257b blocks. In this new scheme for the FlexE frame 50, there is still a round-robin distribution of the 257b blocks to service up to 20 clients (calendar slots), i.e., 0, 1, 2, 3, 4, .... 19, 0, 1, 2, 3, 4, ..., 19, ...

Back in FIG. 5, the diagram for the client distribution is quite similar to that of existing FlexE frame 10, but with 257b block sizes. The 20 clients work out to an even multiple in the existing frame structure. The sequence of 1...20 will be locked with respect to the frame boundaries.

Again, to support the existing FlexE frame 10 in the FlexE frame 50, there is an accumulation of four × 66b blocks and then mapping into a 257b block. This includes accumulation of four × 66b FlexE overhead 12 blocks and insertion into a 257b block for the FlexE overhead 52 with the same occurrence of frame boundaries. This also includes buffering and accumulation of four × 66b calendar slots 16 and insertion into a single 257b block 54. This means each of the data blocks 54 includes four slots 16. This can include a new order for the calendar slots, namely 0000 in a first block, 1111 in a second block, 2222, in a third block, etc. where each block includes four 66b blocks (instances) of the same calendar slot.

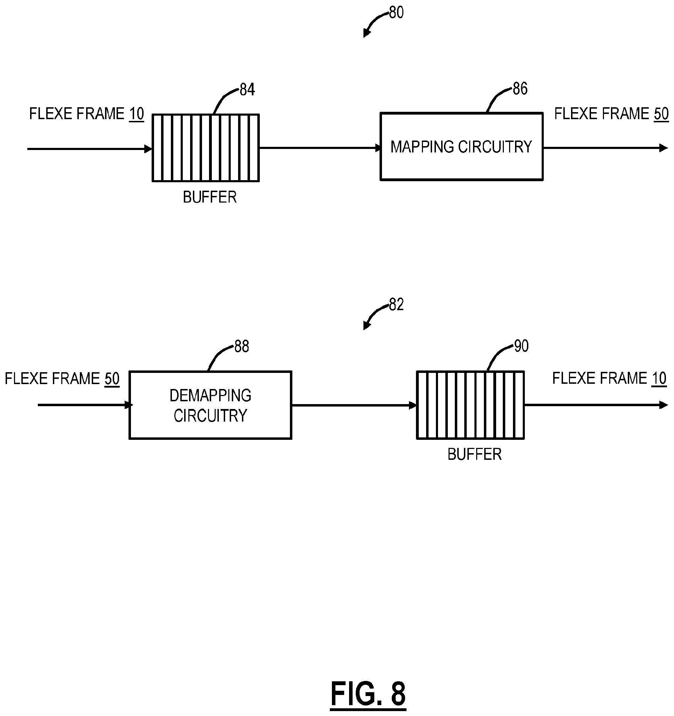

FIG. 8 is a block diagram of a mapping circuit 80 and a demapping circuit 82 for interfacing the FlexE frame 10 at 66b blocks with the FlexE frame 50 at 257b blocks. The mapping circuit 80 receives a FlexE frame 10 and utilizes a buffer 84 to accumulate 66b blocks which are mapped out by mapping circuitry 86 to provide the FlexE frame 50. The mapping circuitry 86 can accumulate four 66b blocks of the same kind, i.e., overhead, a particular calendar slot n where n = 0 - 19.

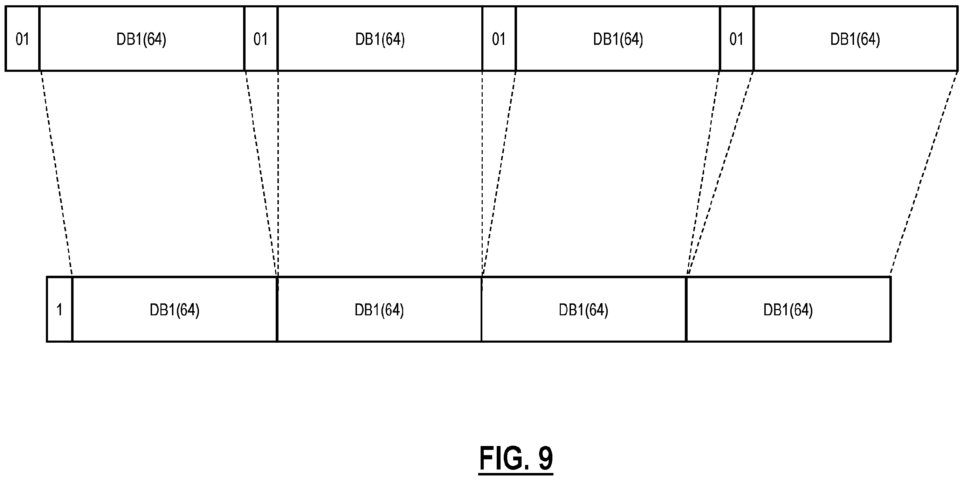

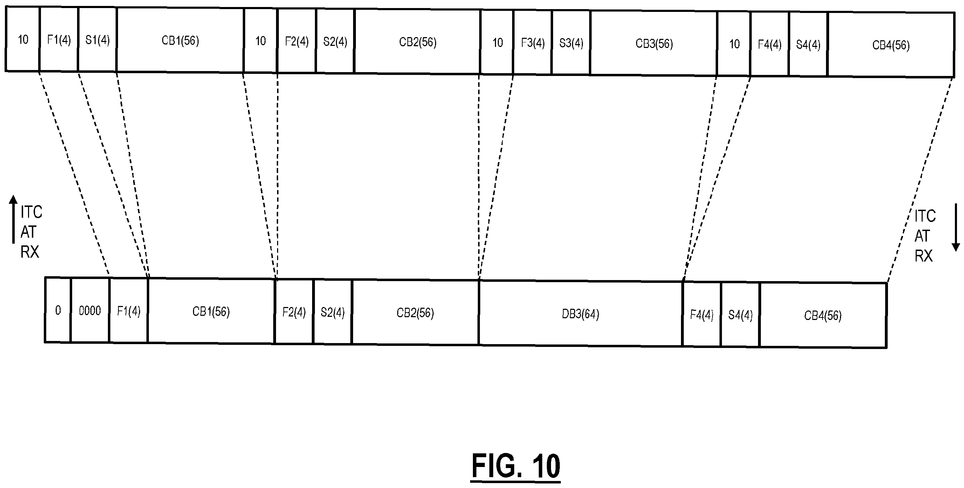

FIG. 9 is a block diagram of mapping four Data Blocks (DB) at 66b into one 257b block. FIG. 10 is a block diagram of mapping four Control Blocks (CB) at 66b into one 257b block. The mapping circuitry 86 can utilize the mappings from FIGS. 9 and 10. A special ordering technique can be defined as follows to convert existing 66b based FlexE frame 10 into this new 257b format. The technique mostly involves reordering the 66b blocks, then reuses standard transcoding method of 4x66b to 257b.

Back in FIG. 8, the demapping circuit 82 receives the FlexE frame 50, and demapping circuit 88 performs an inverse mapping from the mapping circuit 80 and stores the 66b blocks in a buffer 90 where they are read out in a sequence to provide the FlexE frame 10.

From a circuit implementation, this buffering and accumulation will add less than 100ns of latency between the FlexE frame 10 and the FlexE frame 50, while there is an efficient mapping. The FlexE overhead 12 can be reinserted by the demapping circuit 82 or terminated.

Most overhead fields do not have any phase relationship with the data. However, the C (calendar in use) field indicates a swap and this swap is coordinated on both TX and RX so that the add/removal of FlexE clients is hitless. The reordering technique above can buffer data and overhead separately (in the buffers 84, 90) and changes the relationship of the data to the overhead. A pointer (e.g., using reserved FlexE fields in the FlexE overhead 12) can be used to coordinate the event (in relation to the data blocks) when the swap occurs.

In an embodiment, the FlexE frame 50 can originate solely in the 257b domain. An apparatus can include circuitry configured to assemble overhead into a first 257b block of a Flexible Ethernet (FlexE) frame, wherein the overhead includes alignment data, calendar information, and a management channel; circuitry configured to provide a plurality of 257b data blocks according to a calendar and subsequent to the first 257b block with the overhead; and circuitry configured to transmit the FlexE frame have the first 257b block and the plurality of 257b data blocks, wherein the first 257b block and the plurality of 257b data blocks utilize 256b/257b block encoding. Unused 257b data blocks from the plurality of 257b data blocks can be discarded prior to transmission by the circuitry configured to transmit. For example, the 257b FlexE apparatus can be directly connected to a transport system that discards unused 257b blocks to perform a crunch function prior to transport.

## FlexE frame in 66b to 257b process

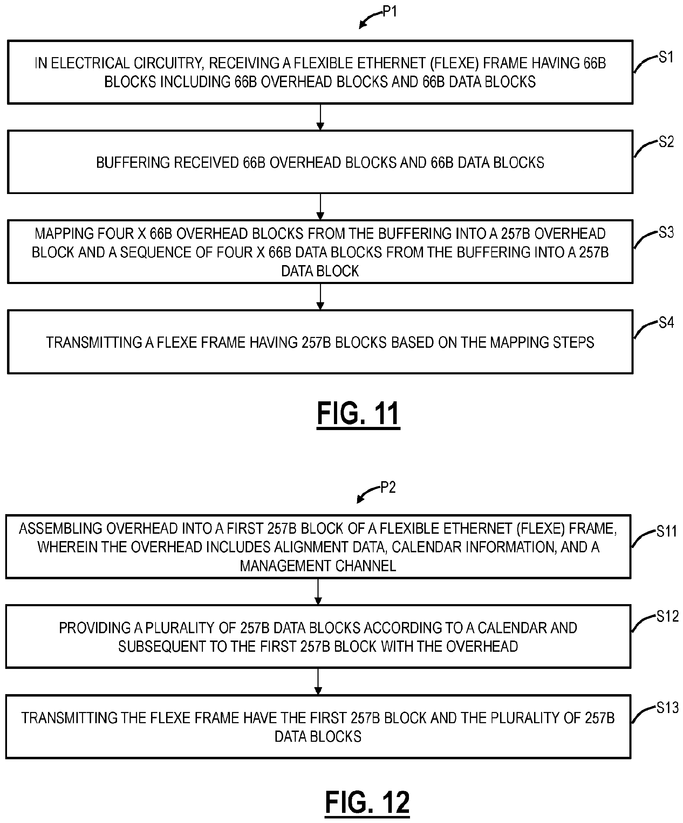

FIG. 11 is a flowchart of a process P1 for mapping the FlexE frame 10 with 66b blocks into the FlexE frame 50 with 257b blocks. The process 100 is implemented in electrical circuitry and includes receiving a Flexible Ethernet (FlexE) frame having 66b blocks including 66b overhead blocks and 66b data blocks (step S1); buffering received 66b overhead blocks and 66b data blocks (step S2); mapping four × 66b overhead blocks from the buffering into a 257b overhead block and a sequence of four × 66b data blocks from the buffering into a 257b data block (step S3); and transmitting a FlexE frame having 257b blocks based on the mapping steps (step S4).

The process 100 can further include receiving a third Flexible Ethernet (FlexE) frame having 257b blocks including 257b overhead blocks and 257b data blocks in a buffer; and demapping the 257b overhead blocks into four × 66b overhead blocks that are stored in the buffer and demapping the 257b data blocks into four x 66b data blocks that are stored in the buffer; and transmitting a fourth FlexE frame having 66b blocks from the buffer.

The mapping can include accumulating four 66b blocks of the same kind from a buffer for mapping into a 257b block, where the same kind is one of overhead and a particular calendar slot n where n = 0 - 19. Both the first FlexE frame and the second FlexE frame can have 20 calendar slots for FlexE client data, wherein each calendar slot in the first FlexE frame can be a 66b block, and wherein the second FlexE frame can have four accumulated calendar slots of the same kind in a 257b block. The sequence can include 1023 repetitions of the 20 four accumulated calendar slots between overhead blocks. Both the first FlexE frame and the second FlexE frame can utilize the same calendar slot distribution scheme. The first FlexE frame can utilize a multiframe approach where each 66b overhead block is one of eight blocks, and wherein the second FlexE frame maintains the multiframe approach where two 257b overhead blocks support the eight blocks. A buffer can separately buffer the 66b overhead blocks and the 66b data blocks. A pointer in the 66b overhead blocks can be utilized to coordinate additional or removal of FlexE clients in the 66b data blocks.

## FlexE frame in 257b

FIG. 12 is a flowchart of a process P1 for mapping the FlexE frame 50 with 257b blocks. The process 200 is implemented in electrical circuitry and includes assembling overhead into a first 257b block of a Flexible Ethernet (FlexE) frame, wherein the overhead includes alignment data, calendar information, and a management channel (step S 11);

providing a plurality of 257b data blocks according to a calendar and subsequent to the first 257b block with the overhead (step S12); and transmitting the FlexE frame have the first 257b block and the plurality of 257b data blocks (step S13), wherein the first 257b block and the plurality of 257b data blocks utilize 256b/257b block encoding. Unused 257b data blocks from the plurality of 257b data blocks can be discarded prior to transmission.

## Switching based on 257b blocks

Switching systems and methods provide switching implementations or algorithms using an Ethernet PCS format based on 257b blocks described herein as a switching technology. Specifically, the systems and methods address 257b blocks in legacy systems (e.g., SONET/SDH), in current systems (e.g., Packet-Optical Transport Systems (P-OTS), OTN, etc.), and in new systems dedicated to 256b/257b switching. With the FlexE switching systems and methods, there are applications (such as intra-data center) that could benefit from layer 1 techniques to bond and possibly switch large (elephant) flows and bypass layer 2 switches. FlexE could be used in certain markets to bypass layer 2 switches, providing cost, power, latency, etc. benefits. It is an objective of the systems and methods described herein to describe circuit switching capabilities with 257b block technologies.

The switching systems and methods can include a 256b/257b block synchronous switch topology and granularity, idle adaptation functionality for adapting phase and frequency in a synchronous network, phase alignment to an Ethernet frame for hitless switching, blocks over fabric links, and the like. The switching systems and methods also include mapping of Ethernet frames into Synchronous Transport System (STS)-n for legacy fabrics, e.g., transcoding of an Ethernet frame to fit in an STS-192. Further, the switching systems and methods provide Ethernet frame switching over OTN over Packet Fabric (OFP) including transcoding an Ethernet frame over OFP for bandwidth savings and using OFP single stream per shim/destination or group.

The switching systems and methods provide various implementations and related ideas for TDM switching with an Ethernet frame format. As described above, the FlexE implementation agreement (in OIF) defines a TDM frame structure using 20x calendar slots per 100G group. These include block streams distributed in a round-robin fashion to create a TDM structure. The switching systems and methods deal with mapping such 256b/257b block streams into STS-n structures, to be switched using legacy systems and fabrics. This can include mapping 256b/257b into STS using asynchronous stuffing, mitigating against skew across a fabric, and performing frequency and phase alignment for the 256b/257b into a synchronous system. Some transcoding techniques can be applied to lower bandwidth and fit into an STS-n where n is a standard number (such as STS-192).

Also, the switching systems and methods deal with mapping 256b/257b blocks into existing P-OTS cell switch architectures. The TDM slots are SARed (Segmentation and Reassembly) using OIF OTN over Packet (OFP ) techniques and can be switched as a TDM stream across existing fabrics, co-locating packet and OTN traffic. OIF OFP is described in IA # OIF-OFP-01.0, "OTN Over Packet Fabric Protocol (OFP) Implementation Agreement" (November 2011). FlexE is beyond the scope of IA # OIF-OFP-01.0, but uses similar concepts. Timing transparency can be used for transparent switching of the 256b/257b streams.

Finally, the switching systems and methods deal with possible new TDM fabric architectures optimized around switching 256b/257b blocks and streams. The switching systems and methods can be used to augment existing switch fabrics, P-OTS and OTN platforms. Further, the switching systems and methods can be used in next-generation switches, such as for use in applications which have not adopted OTN to date (e.g., intra data center applications).

## 257b block-based switching fabric

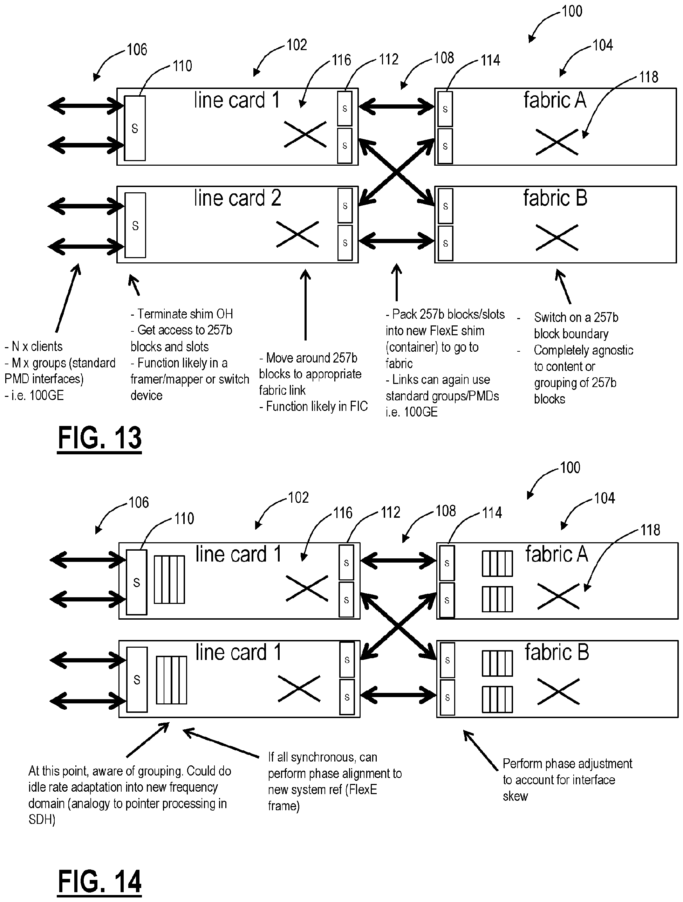

FIGS. 13 and 14 are block diagrams of an Ethernet frame switch 100 showing FlexE transparent (FIG. 13) and showing FlexE termination (FIG. 14). The FlexE switch 100 is a new fabric, optimized for switching, i.e., the switch 100 is not a SONET/SDH STS switch, a P-OTS or OTN switch. For illustration purposes, the switch 100 is illustrated with two line cards 102 and two fabrics 104. In FIG. 13, the line cards 102 include ingress/egress ports 106 which can be communicatively coupled externally to the switch 100. The line cards 102 connect to one another via the fabrics 104 through interfaces 108, such as a backplane, midplane, etc. In an embodiment, the switch 100 is a Clos-based switch between the line cards 102 and the fabrics 104. In physical implementations, the line cards 102 and the fabrics 104 include circuitry for implementing the functions described herein, and the ports 106 and the interfaces 108 are interconnection mechanisms.

Those of ordinary skill in the art will recognize the switch 100 can include other components which are omitted for illustration purposes, and that the systems and methods described herein are contemplated for use with a plurality of different hardware configurations with the switch 100 presented as an example. For example, in another embodiment, the switch 100 may not include the fabrics 104, but rather have the corresponding functionality in the line cards 102 (or some equivalent) in a distributed fashion. For the switch 100, other architectures providing ingress, egress, and switching are also contemplated for the systems and methods described herein.

The ports 106 can include N x clients over M x groups, with standard PMD interfaces (e.g., 100GbE). The line cards 102 can include a shim termination block 110, facing the ports 106, and a shim termination block 112, facing the interfaces 108. The fabrics 104 can include a shim termination block 114, facing the interfaces 108 and a FlexE shim termination block 112. The shim termination blocks 110, 112, 114 can include circuitry to perform the following functions.

The shim termination block 110 is configured to terminate shim overhead and to provide access to 256b/257b blocks and slots. The shim termination block 110 functions similar to a framer/mapper in a switch device. The line cards 102 include switching circuitry 116 which can operate as a first stage and a third stage (or ingress stage and egress stage) in a Clos-architecture. The switching circuitry 116 is configured to move around (switch) 256b/257b blocks to an appropriate fabric link, on the interfaces 108. The switching circuitry 116 can operate similarly in a Fabric Interface Controller (FIC).

On the interfaces 108, the shim termination blocks 112, 114 pack 256b/257b blocks into a new shim (container) to go to the fabric. The links on the interfaces 108 can use standard groups/PMDs, such as in a Chip-to-Chip (C2C) application. The fabrics 104 include switching circuitry 118 which can operate as a second stage (or center stage) in the Clos-architecture. The switching circuitry 118 is configured to switch on a 256b/257b block boundary and is completely agnostic to the content or grouping of the 256b/257b blocks.

The switch 100 utilizes a synchronous scheme. However some idle adaptation (part of full shim function and based on 802.3 clause 82) could be performed at entry/exit of the line card 102. The fabrics 104 can utilize frequency and time (phase and frequency) synchronization (to keep proper order and handle groups of slots/blocks). The time synchronization could be based on frame boundaries, i.e., an analogy to 8k from SDH. This could allow hitless switching given that all flows are aligned.

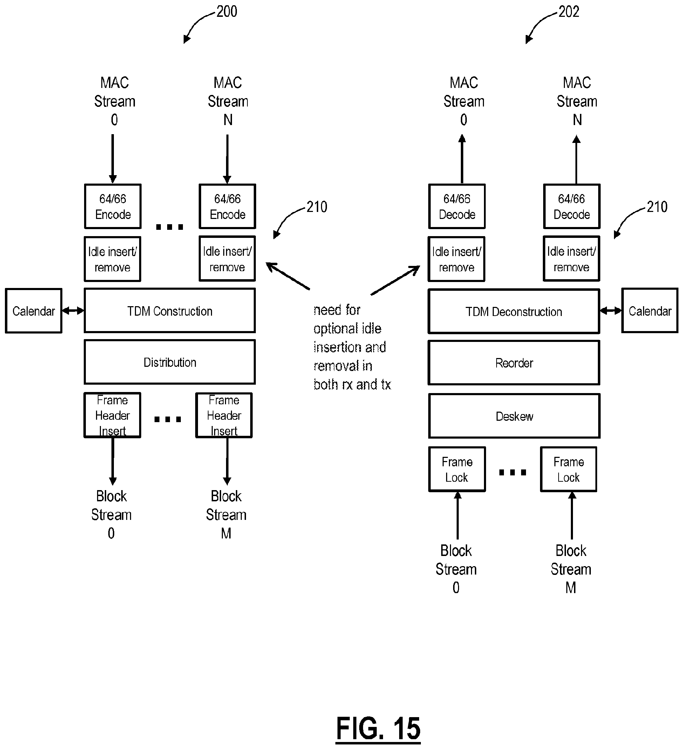

FIG. 15 is a block diagram of processing performed by the line cards 102 at the ports 106. Specifically, FIG. 15 includes ingress processing 200 and egress processing 202. The switch 100 can include idle adaptation 210 based on 802.3 clause 82 scheme, which is analogous to pointer processing performed in SONET/SDH. The idle adaptation 210 could be done at ingress/egress of the switch 100, i.e., at the ports 106, and could be part of the shim function, i.e., the shim termination block 110.

The ingress processing 200 is configured to take N MAC streams and output M block streams. The N MAC streams are 256b/257b encoded, the idle adaptation 210 is performed, i.e., idle insert/remove based on clause 82, TDM construction is performed with the calendar, distribution is performed, and a frame header is inserted into the M block streams. The egress processing 202 is configured to take M block streams and output N MAC streams. The M block streams are frame locked, deskewed, reordered, TDM deconstructed with the calendar, the idle adaptation 210 is performed, again based on clause 82, i.e., idle insert/remove, and 256b/257b decoded.

**Switching using a SONET/SDH STS fabric**

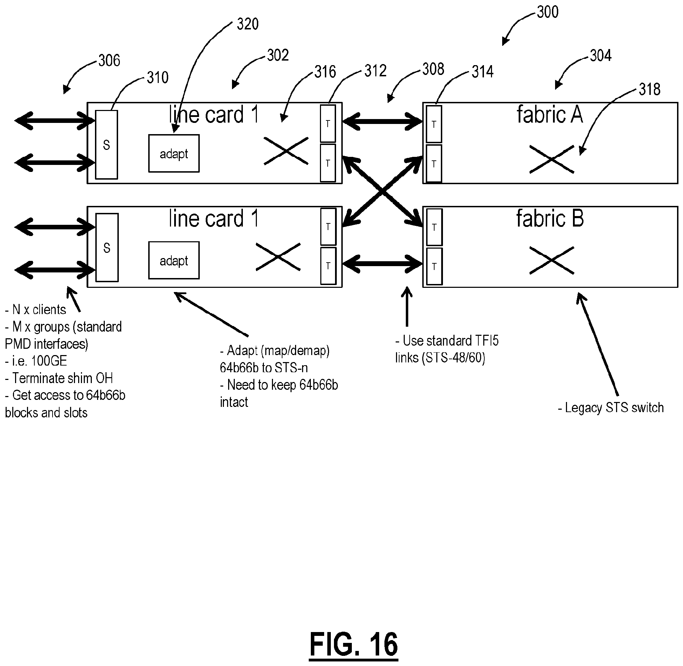

FIG. 16 is a block diagram of an STS switch 300 adapted to support Ethernet frames. The STS switch 300 is a TDM fabric that supports SDH/SONET switching at the STS-1 level and a TDM Fabric with Frame Interface (TFI). The STS switch 300 is adapted to support further Ethernet frame switching. The STS switch 300 includes line cards 302, fabrics 304, ports 306, and interfaces 308. The line cards 302 include a shim termination block 310 and a TFI block 312 and the fabrics 304 include a TFI block 314. Also, the line cards 302 include switching circuitry 316 and the fabrics 304 include switching circuitry 318.

The STS switch 300 is configured to adapt 256b/257b blocks into STS-n. The rate of the calendar slots can be (100GE / 20 - (20*1024)/(1+20*1024)) = 5.15G which requires an STS-100 container for 64b/66b. The STS switch 300 is configured to transcode 64b/66b into 256b/257b (from IEEE 802.3bj), specifically 5.15G * 32/33 * 257/256 = 5.013G, which requires an STS-97. The STS adaptation can implement various "tricks" or the like to fit into the 64b/66b blocks into less spaces, such as to reuse a part of the STS and use the idle adaptation to get to STS-96, which is a meaningful number in SONET/SDH. Since the minimum FlexE client granularity is 10G, it is possible to group 2x slots and map into an STS-192c. The STS switch 200 can use a special 256b/257b "O code," i.e., 0x9, as stuffing to fit 256b/257b stream into the STS-n frame format. The STS switch 300 can phase align Ethernet frame boundaries to SDH 8k to preserve 256b/257b block skew for groups of calendar slots.

At the ports 306 and the shim termination block 310, there are N x clients and M x groups (which can include standard PMD interfaces). The shim termination block 310 terminates the shim overhead and provides access to the 256b/257b blocks and slots. The STS switch 300 includes adaptation circuitry 320. The adaptation circuitry 320 is configured to adapt (map/demap) 256b/257b to STS-n while keeping bit transparency for the 256b/257b stream. Subsequent to the adaptation circuitry 320, the STS switch 300 provides STS switching. Specifically, the switching circuitry 316, 318 provides STS level switching and the TFI blocks 312, 314 utilize standard TFI5 links.

**Switching using OFP**

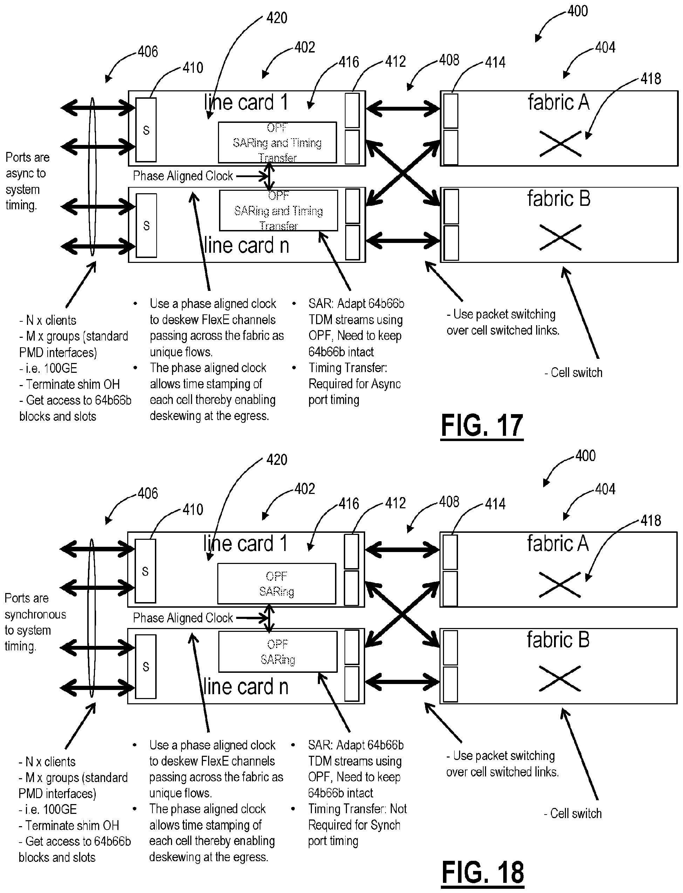

FIGS. 17 and 18 are block diagrams illustrate an OFP switch 400 adapted to support Ethernet frame switching. Note, OFP is described in IA # OIF-OFP-01.0 "OTN Over Packet Fabric Protocol (OFP) Implementation Agreement" (November 2011). The objectives of the OFP switch 400, for FlexE switching, include maintaining 256b/257b coding; switching in N x 5G, 20G, or 25G units, i.e., a single 5G, 20G, or 25G calendar slots or a full client flow; maximizing fabric bandwidth efficiency (by minimizing header and/or payload); providing timing transparency using B+/-1; performing byte alignment of payload units for ease of re-creating a stream; minimizing fabric related skew between bonded flows; and performing error detection across fabric between SARs. It may be assumed that the fabric packets are not required to carry an integral number of payload units, and the fabric maintains packet order within a flow.

An OFP header includes 32 bits, as follows: Timestamp (16), Reserved (6), Sequence Number (2), Previous Packet Size Indicator 1 (2), PPSI2/BIP-8 (2), CSI (3), Parity (1). The OFP switch 400 can use the OFP header in part for switching. The Timestamp is needed for fabric latency variation compensation, and there could possibly be a reduction in resolution by 1 or 2 bits. The Reserved bits are available to be used. The Sequence Number is needed for lost packet detection; the Previous Packet Size Indicator 1 provides timing transfer lost packet compensation. The PPSI2 is not needed unless the double packet loss probability is high enough - not likely, but the BIP-8 is desirable to provide SAR-to-SAR error detection; could be added to OFP IA. The CSI is optional and is provided for fast protection switching. So, with changes, there could be 6 or as many as 9-11 bits available in the OFP header.

Timing transparency is required when a destination client is not synchronous with a source client. OFP varies packet size from nominal by +/- one byte to signal the source rate. This requires a Packet Size Decision (PSD) at both ingress and egress points and uses PPSI to communicate previous packet size to allow for single packet loss. For rate adaptation, the fabric has speedup over the line, so idle removal on ingress is not required, and the egress point is the logical place for idle insertion/removal. If timing transparency is not required, fabric packets could have a fixed payload size. It is possible to make packet size a fixed multiple (likely fractional) of a payload unit. A fixed pattern could be aligned to SQ, additional header bit(s) if necessary.

The OFP switch 400 can use OFP mechanisms for the cell switch fabrics. This can include carrying the complete 257b block in the packet payload; this reduces header requirements; every 8th block is byte aligned; need to signal block alignment. Block alignment signaling can be serialized since it is used only at traffic (re)start; reduces header requirements; applies to all three options above. The block pointer can be serialized using one or more header bits, aligned to SQ. For example, a possible bit sequence for a 5-bit offset serialized using a single header bit over 8 packets: starting on SQ=0, 0, offset[0], offset[1], offset[2], 1, offset[3], offset[4], unused. This can be applied to any of the preceding options that require block alignment signaling, not just the 257B option in this clause.

For skew management, OFP provides a fixed fabric delay, greater than max fabric delay, to smooth out fabric packet delay variations. All fabric switched flows can be configured to have the same delay, with sub-microsecond precision (typically sub 100ns).

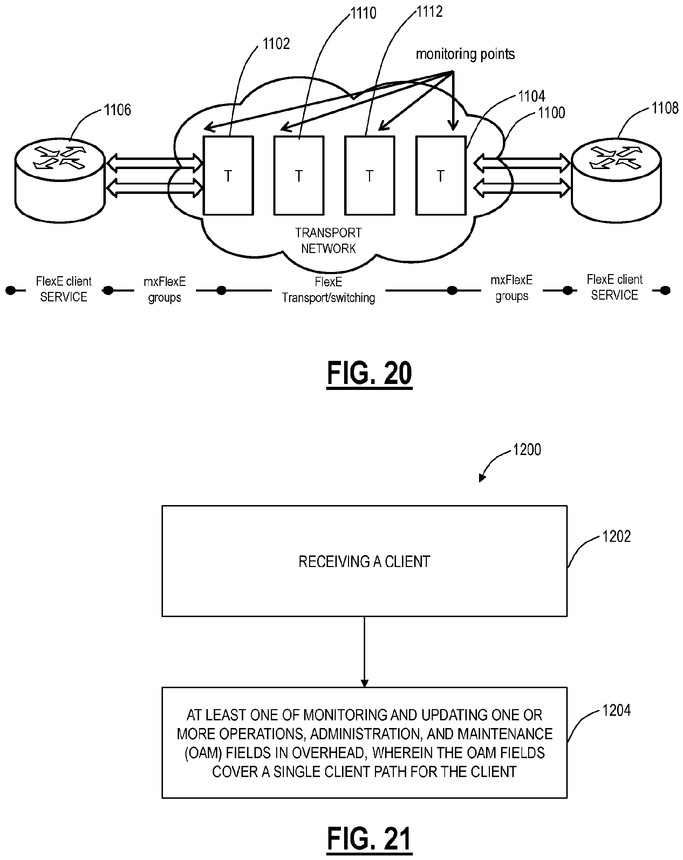

The OFP switch 400 in FIGS. 17 and 20, similar to the switch 100 and the STS switch 300, includes line cards 402, fabrics 404, ports 406, and interfaces 408. The line cards 402 include a shim termination block 410 and a block 412 and the fabrics 404 include a block 414 which physically forms the interfaces 408 with the block 412. Also, the line cards 402 include circuitry 416 and the fabrics 404 include switching circuitry 418.

FIG. 17 illustrates the OFP switch 400 for asynchronous ports. Here, the ports 406 are asynchronous to system timing. FIG. 18 illustrates the OFP switch 400 for synchronous ports. Here, the ports 406 are synchronous to the system timing. The ports 406 supports N x clients or M x groups (with standard PMD interfaces). The FlexE shim termination block 410 is configured to terminate shim overhead and provide access to 64b/66b blocks and slots. The line cards 402 use a phase aligned clock to deskew channels passing across the fabric 404 as unique flows. The phase aligned clock allows time stamping of each cell thereby enabling deskewing at the egress.

In FIG. 17, the circuitry 416 performs OFP SARing and Timing Transfer and, in FIG. 21, the circuitry 416 performs OFP SARing. The SAR function adapts 256b/257b TDM streams using OFP, keeping 256b/257b blocks and streams intact. In FIG. 17, the timing transfer is required for asynchronous port timing, but not required in FIG. 18. The switching circuitry 418 uses cells for OTN TDM switching, e.g., using OIF OFP specifications. The OFP switch 400 operates creating cell flows based on 256b/257b calendar slots.

The OFP implementation can support a single fabric channel for each client. This would require the merging of data from each channel into a single fabric channel. This scheme would reduce connection scaling resource demand in the fabric system assuming that the average client had a bandwidth greater that a single calendar slot. If the client calendar slots were distributed across multiple ingress cards we could support a multiple fabric channels (1 from each card/client) utilizing the phase aligned clock to allow for the mapping into of the egress channels.

The OFP can also support a single fabric channel for each calendar slot. This simplifies the implementation at the expense of connection scaling in the fabric. It does require the support of the phase aligned clock to ensure channel alignment at the egress.

**Service CAM**

OAM systems and methods are described to extend and use Ethernet frame clients as a maintenance entity and service with defined OAM functionality. The OAM systems and methods described various different approaches to an OAM layer in client overhead. The OAM systems and methods also address transport performance monitoring complexities including error checking allowing monitoring and fault isolation of clients, without having to perform packet-level error checking (i.e., MAC Frame Check Sequence (FCS)). There are markets (such as intra data-center) that could benefit from layer 1 techniques to bond, possibly switch large (elephant) flows and bypass layer 2 switches. These markets have not adopted OTN for such applications. Ethernet frame formats could be used in such markets to bypass layer 2 switches, providing cost, power, latency, etc. benefits. Adding client OAM will enable such application using client as a service in a provider's network, and it will help with fault isolation and error checking of variable number/size of flows.

Advantageously, the OAM systems and method augment/create the concept/application of a client service with simplified performance monitoring for transport equipment. The OAM systems and methods add OAM fields to the client, including new Local Fault (LF)/Remote Fault (RF) client service fields independent of the group, which is tied to PHY LF/RF. The Client OAM provides fields in the frame overhead and/or in the path Reconciliation Sublayer (RS); two approaches are considered.

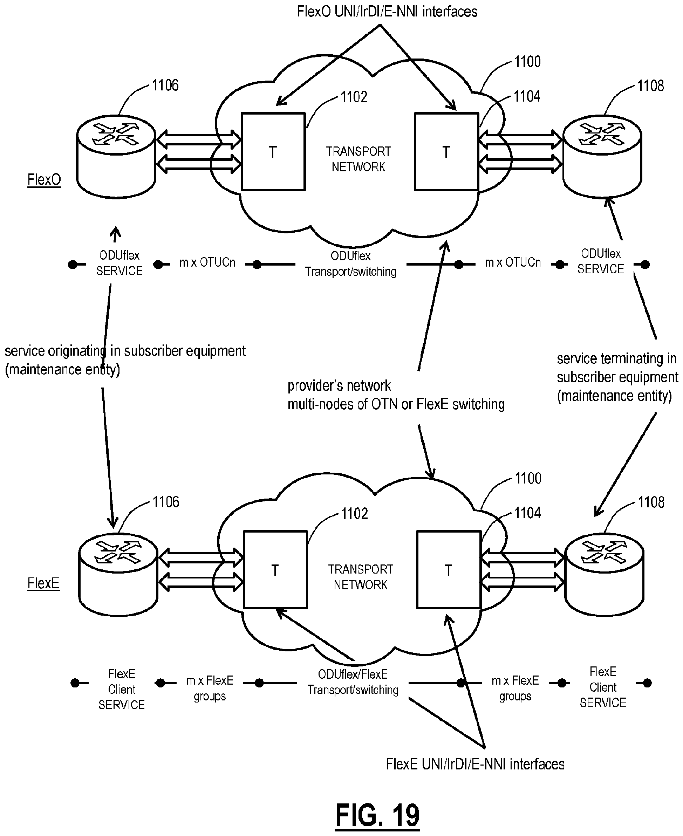

FIG. 19 is a network diagram illustrates a comparison of FlexO service OAM with the FlexE Client service OAM systems and methods described herein. Specifically, the same equipment is shown in FIG. 19 for FlexO and FlexE, including a transport network 1100 with network elements 1102, 1104 coupled to switches/routers 1106, 1108. In an embodiment, the OAM systems and methods enable an Ethernet frame client to be transported as a service (or private line service) in a provider network. The switches/routers 1106, 1108 can be referred to as subscriber equipment where a service originates or terminates, i.e., a maintenance entity. In FlexO, the service can be an ODUflex, and, in FlexE, the service can be a FlexE client. The network elements 1102, 1104 are communicatively coupled to the network elements 1102, 1104. In FlexO, the interconnection between the network elements 1102, 1104 and the switches/routers 1106, 1108, respectively, can be m x OTUCn, and, in FlexE, the interconnection can be m x FlexE groups.

The transport network 1100 can include a plurality of network elements in addition to the network elements 1102, 1104. The various network elements in the transport network 1100 are configured to perform transport/switching. In FlexO, the transport/switching can be at ODUk/flex or some other variant of OTN. In FlexE, the transport/switching can be ODUk/flex and/or FlexE-based switching, as described herein. In FlexO, the network elements 1102, 1104 provide User-Network Interface (UNI)/Inter-Domain Interface (IrDI)/External Network-Network (E-NNI) interfaces. Conversely, the OAM systems and methods enable FlexE UNI/IrDI interfaces at the network elements 1102, 1104.

In an embodiment, the network 1100 can support a client, and the network 100 can include a plurality of nodes interconnected to one another, wherein the client is configured between two nodes; wherein each of the two nodes include circuitry configured to receive the FlexE client, and circuitry configured to update or monitor one or more Operations, Administration, and Maintenance (OAM) fields in FlexE overhead, wherein the OAM fields cover a single client path for the FlexE client. One of the two nodes can be a service terminating subscriber equipment and another of the two nodes can be a network element in a transport network, wherein the FlexE client can include a User-Network Interface (UNI). Each of the two nodes can be a network element in a transport network, and each of the two nodes can be configured to operate as an OAM monitoring point for the FlexE client in the transport network. The one or more OAM fields can include any of a client service monitoring field, a connectivity/trace, link fault, remote fault, maintenance states, test signals, link neighbor discovery, and backwards fault and error indication.

**FlexE Client Performance Monitoring**

FIG. 20 is a network diagram of a network with various client OAM monitoring points. In FIG. 20, the transport network 1100 is illustrated with additional network elements 1110, 1112, and each of the network elements 1102, 1104, 1110, 1112 can be configured as a monitoring point for service OAM. In Ethernet, current transport equipment typically relies on MAC CRC (layer 2) for fault isolation and performance monitoring. With FlexE, this becomes complicated as FlexE introduces a variable amount of clients on a single/multiple ports (groups) and a variable rate for the clients (Flexible MAC). The client OAM systems and methods can include a simpler FlexE client service monitoring field (e.g., Bit Interleaved Parity 8 (BIP8) or the like) that covers only the 256b/257b codes for a single client path, i.e., a single error check per client, instead of CRC at the packet level. This can be viewed analogously to the PM BIP8 that covers an ODUflex service path. Such an approach with a FlexE client service monitoring field simplifies monitoring and fault isolation. Nodes in a transport network could be 3R regens (i.e. Regenerator Section (RS)-OTN) or switches (i.e. Multiplex Section (MS)-OTN or FlexE switches).

**FlexE OAM Fields**

The following table includes proposed OAM fields:

| |
|---|
| Bit Error Check (e.g., BIP8) |
| Connectivity/Trace (e.g., Trail Trace Identifier (TTI)) |
| Link Neighbor Discovery (i.e. LLDP) |
| Link Fault |
| Remote Fault |
| Maintenance States (e.g., locked, loop-back, test) |
| Test signals (e.g., Pseudorandom binary sequence (PRBS)) for turn-up |
| Backwards fault and error indication |

The FlexE OAM cannot reuse the group Remote Fault (RF)/Local Fault (LF) for clients as this would flag group/PHY faults in the transport equipment. Instead, the FlexE OAM could define a new "O code" (e.g., 0x9) for client RF/LF to disassociate from the group/PHY. Again, drawing analogies to the OTN implementations, this is analogous to LO ODUk-AIS Path Monitoring (PM) relative to LO/HO Section Monitoring (SM).

In an embodiment, OAM fields are included in the Ethernet Time Division Multiplexing (TDM) frame overhead. The TDM frame overhead has a portion for all calendar slots and paths. For example, the client OAM could occupy some or all of the reserved portions of the FlexE TDM frame overhead. However, it might be tight fitting OAM overhead for up to 10 clients (per group) in these portions. As such, an option could be to multiframe to OAM overhead, analogous to what OTN does with the HO OPUk path OH. The FlexE OAM could be switched along with the calendar slots to provide a path. Another option could include reducing the management channel to provide more room for the Client OAM or reusing the management channel capacity with a messaged based OAM approach on a general communications channel.

In another embodiment, a new "O" code for implementing FlexE OAM at the RS layer. This exemplary embodiment includes injecting a new "O" code (e.g., 0x9), 1/16k occurrence and steal bandwidth of the client (e.g., 59 parts per million (ppm)) - worst case standard Ethernet at 100G has 400ppm of idles in there. This can be used for a new client LF/RF and to differentiate from group LF/RF (0x0), and this can include specific OAM per client stream, which is then mapped to calendar slots along with the client stream. These OAM fields are tied to FlexE clients and not calendar slots. This second option is analogous to LO ODUk PM overhead.

**FlexE monitoring process**

FIG. 21 is a flowchart of a process 1200, implemented in a node, for monitoring a client service that uses a 257b block-based frame in a network. For example, the node can include the network elements 1102, 1104 or the switches/routers 1106, 1108. The process 1200 includes receiving a client (step 1202); and at least one of monitoring and updating one or more Operations, Administration, and Maintenance (OAM) fields in overhead, wherein the OAM fields cover a single client path for the client (step 1204). The one or more OAM fields can include a client service monitoring field which does not rely on Cyclic Redundancy Check (CRC). The one or more OAM fields can include a client service monitoring field including a Bit Interleaved Parity (BIP) code. The one or more OAM fields can include any of a connectivity/trace, link fault, remote fault, maintenance states, test signals, and backwards fault and error indication. The one or more OAM fields can be disassociated from Local Fault and Remote Fault information for an associated group/PHY. The one or more OAM fields can be located in the overhead, possibly using a multiframe scheme. The one or more OAM fields can be implemented in a Reconciliation Sublayer with a new associated Operational code. The node can be one of a Regenerator Section (RS) Optical Transport Network (OTN) switch, a Multiplex Section (MS) OTN switch, and a native switch.

**FlexE Multi-Service**

In various embodiments, multi-service and timing transparency systems and methods are described. The systems and methods introduce timing transparency for clients or other client types. A new stuffing block (O code) can be used to perform rate adaptation and timing information (Cn) and can be sent in overhead. Timing transparency enables multi-service applications where the Ethernet frame can be used for aggregation and transport/switching of different types of clients (e.g., FC, Ethernet, OTN, Common Public Radio Interface (CPRI), and the like). An objective of the systems and methods is to provide capabilities to address OTN type of applications. Thus, FlexE, MTN, etc. could actually be used as the next B100G container in ITU standardization. An analogy can be made to current B100G OTN/FlexO, where the High Order Optical channel Transport Unit Level Cn (OTUCn) is the FlexE n x group/PMD and the Optical channel Data Unit Flex (ODUflex) is like the FlexE client (ETC in G.8010 notation). The systems and methods described herein propose to also map the ODUflex into FlexE shim as a client, or the like.

Ethernet is driving volumes for modules and components due to its widespread adoption for a variety of applications. Many believe that a major disadvantage to OTN is the cost mostly driven by relatively small market volumes. It is unlikely that modules (i.e. CFP2, QSFP28) will be purposely built for OTN, and this is why OTN is using schemes for adapting to a similar structure to Ethernet PMD, in order to enable reuse of modules and components (possibly clocked a bit higher). A good example of this in the past was Optical Transport Lane (OTL) adaptation to align with 100 Gigabit Attachment Unit Interface (CAUI) lane structures and bit interleaving. Aligning completely to an Ethernet PMD/PHY by using the FlexE group + shim as a High Order (HO) container and section layer is advantageous. For example, this could be a competing approach to B100G OTUCn. Also, being able to support timing transparency enables applications of Synchronous Ethernet (SyncE) over FlexE in port expander and access aggregation applications. The systems and methods described herein use FlexE as the next B100G HO container and section layer.

Variously, the systems and methods include a new "O" code in 256b/257b stream to stuff into the shim, a scheme for timing transparency, ODUk adaptation for a next-gen B100G section, adaptation of multi-service (e.g., CPRI, FC, etc.) into FlexE, a FlexE hierarchy, support for SyncE over the FlexE shim, and a new client type field in the FlexE overhead. The systems and methods described herein describe mapping other 256b/257b clients into a FlexE TDM structure, to expand the reach and application.

### FlexE timing transparency

One requirement of FlexE multi-service is timing transparency of clients when mapped to the shim. There is no removal of idles, so the group might have to run faster, e.g., 50ppm to leave room for the shim overhead. This also enables application of having clients being SyncE and Precision Time Protocol (PTP) (IEEE 1588) capable. The systems and methods propose a new "O" code (e.g., 0x9) to stuff and rate adapt 256b/257b client streams into the calendar slots.

The FlexE group (e.g., 100GbE PMD) is the timing reference. The systems and methods transmit a timing value, with a set bit resolution Cn (e.g., 24 bits) in the overhead. This could be multiframe to have Cn timing information per overhead for a given calendar slot or client. This is analogous to the Generic Mapping Procedure (GMP) process in OTN, except stuffing and timing information is transmitted separately, but long-term average equal.

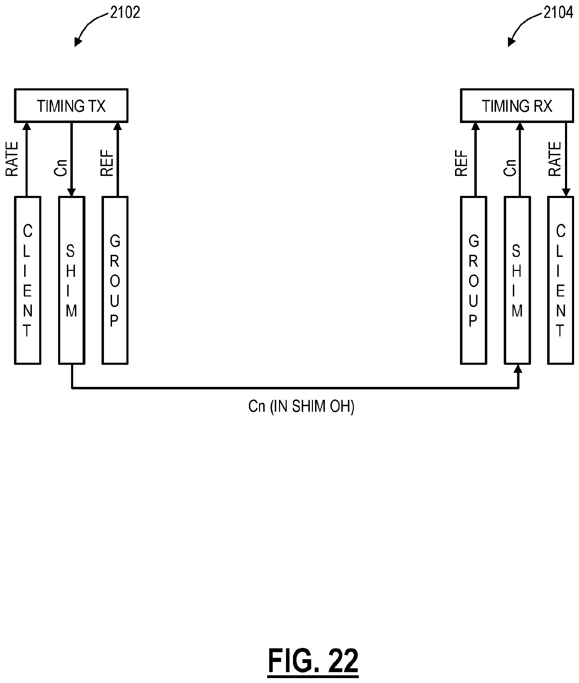

FIG. 22 is a diagram of a differential timing process between two nodes 2102, 2104 utilizing FlexE. At the transmitting node 2102, a client provides a rate for the timing TX and a group provides a timing reference (REF), and the flexE shim provides Cn in the shim overhead to the receiving node 2104. The FlexE shim at the node 2104 provides Cn to the timing RX, as well as the FlexE group providing a timing reference and the timing RX providing the FlexE client a rate.

### Multi-service mapping process

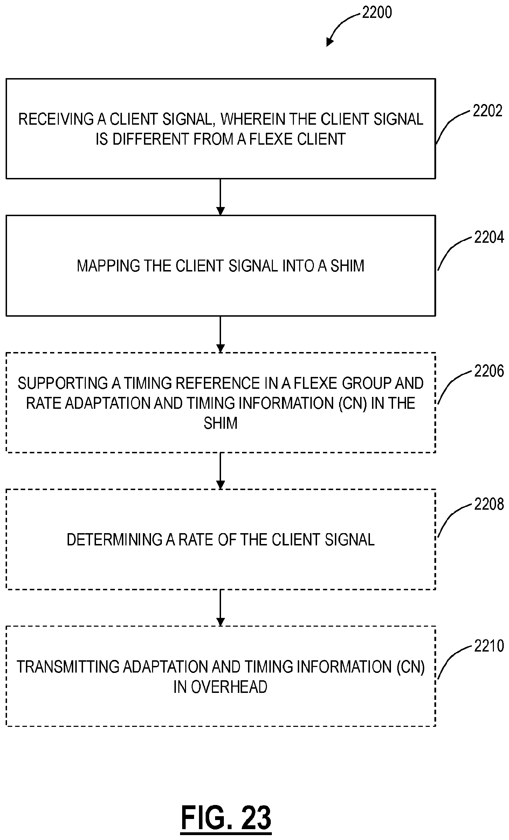

FIG. 23 is a flowchart of a process 2200, implemented in a node, for supporting multi-service with Flexible Ethernet (FlexE), MTN, etc. For example, the node can include a network element, a switch, a router, etc. The process 2200 includes receiving a client signal, wherein the client signal is different from a client (step 2202); and mapping the client signal into a shim (2204). The process 200 can further include supporting a timing reference in a FlexE group and rate adaptation and timing information (Cn) in the shim (step 2206); and determining a rate of the client signal (step 2208). The process 2200 can further include transmitting adaptation and timing information (Cn) in FlexE overhead (step 2210). The client signal can utilize 256b.257b encoding. The client signal can be one of Fibre Channel, Infiniband, Optical Transport Network (OTN), Common Public Radio Interface (CPRI), and Synchronous Optical Network (SONET) first mapped into OTN. The mapping step 2204 can further include inserting client information associated with the client signal in overhead. The shim can be a first shim, and wherein the client signal can include a second shim. The mapping step 2204 can further include using an Operational code to stuff and rate adapt the client signal.

### FlexE C2C interfaces

FlexE is defined for system/network IrDI/UNI/E-NNI interfaces. Historically, OIF has been defining C2C interfaces, such as Serdes Framer Interface Level 5 (SFI-5), but OIF has recently stopped this practice. IEEE and ITU have developed their own system interfaces (OTL, CAUI), which have seen adoption for C2C applications. Interlaken is currently used for C2C packet interfaces, but is complicated and is constrained to C2C applications (within a system).

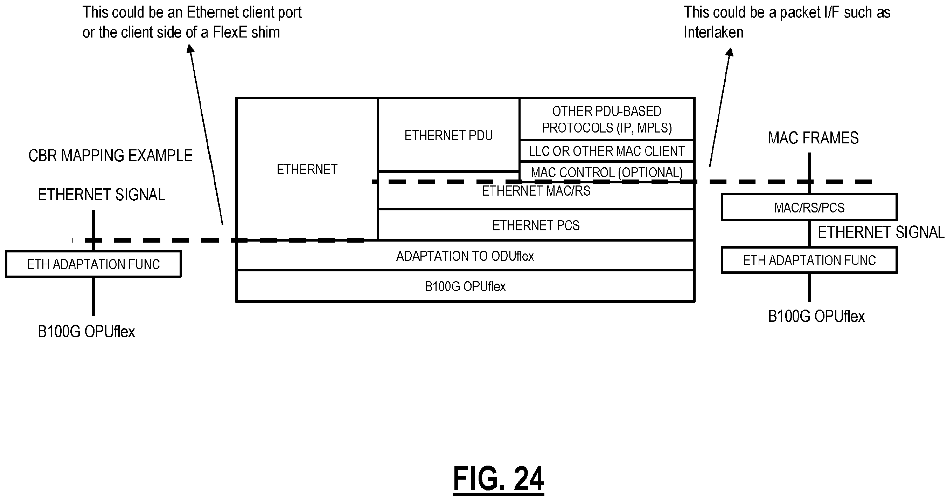

FIG. 24 is a logical diagram of a protocol stack for Layers 1-3. ITU group is aligning packet mapping and FlexE client mapping. A Chip-to-Module (C2M) interface from an IrDI is similar to a C2C interface from an NPU, i.e., similar logic and similar interface. Interlaken has been used historically for packet interfaces from NPU. FlexE already provides channelization, but is limited to fixed sizes and minimum granularity of 10G.

The protocol stack illustrates two example mappings - a Constant Bit Rate mapping (CBR) example and a Packet Data Unit (PDU) mapping example. In the CBR example, an ETH adaptation function is used to map an Ethernet signal to a B100G OPUflex. This could be an Ethernet client port of the client side of a FlexE shim. In the PDU example, MAC frames are processed by a MAC/RS/PCS layer to provide an Ethernet signal which is adapted by an ETH adaptation function to a B100G OPUflex. A packet from an Interlaken interface is similar to a FlexE client packet from a FlexE interface.

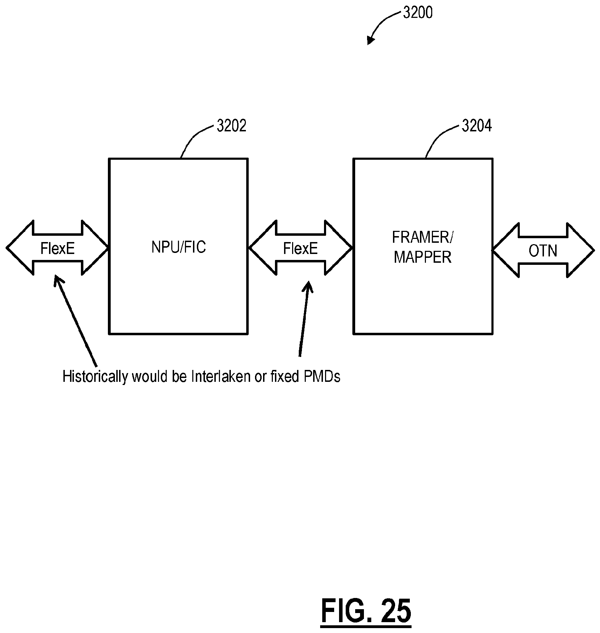

FIG. 25 is a logical diagram of use of FlexE in a C2C application 3200. The C2C application 3200 includes an NPU or Fabric Interface Chip (FIC) 3202 coupled to a framer/mapper device 3204. The interfaces to/from the NPU/FIC 3202 can be FlexE or a stream of 257b blocks in the C2C application 3200, specifically FlexE between the NPU/FIC 3202 and the framer/mapper 3204. The interface from the framer/mapper 3204 can be OTN. Note, historically, the FlexE interfaces in the C2C application 3200 were Interlaken.

The C2C application 3200 provides a simplified framer/mapper 3204 and NPU/FIC 3202 design by supporting less interfaces. Normal Ethernet interfaces are not channelized at L1 (need to rely on Virtual Local Area Network (VLAN) or other L2/L3 channels). Extended X Attachment Unit Interface (eXAUI) attempted introducing channelization to 10GbE with header extensions. Normal Ethernet interfaces need to handle subrate with PAUSE frames. Ethernet PMDs (i.e. KR4) already support FEC, however the Interlaken Alliance is currently evaluation FEC schemes.

To make a FlexE interface suitable as a C2C packet interface, the systems and methods described herein include flow control, enhanced channelization, and optionally mapping OTN to FlexE. Interlaken historically runs faster to carry channelization, flow-control and other bits of information. FlexE for C2C could also run faster to account for added information bits, or more groups can be added to the FlexE shim to account for overclocking.

## FlexE C2C interfaces - channelization

FlexE already provides calendar slots and channelization with granularity of 5G, and a minimum client rate of 10G. This is limited for applications requiring more than 20x channels and client rates <5G (such as 1Gbe). As such, the systems and methods propose the concept of a logical channel in FlexE that can share single or grouped calendar slots. The logical channel can change on frame boundaries and is more flexible than a calendar. These logical channels can be bursts of 20k blocks (matching frame header) or 80k blocks (multiframe). The logical channels can be identified through a logical channel number for a following period in the FlexE overhead. These logical channels are not limited in granularity

For example, the logical channel number can be in the FlexE reserved area or in bits from the management channel (which is not needed for C2C applications) - limited in number of bits. In another exemplary embodiment, the logical channel number could be identified in the FlexE calendar area, i.e., repurpose the calendar area for logical channels, more dynamic behavior. In a further exemplary embodiment, another "O" code (operational code) could be defined or it could be in the data block immediately following a frame header (signified with "O" code 0x5). The "O" codes could also be distributed and not aligned to FlexE frame boundaries.

## FlexE C2C interfaces - flow control

Flow control can use a 20-bit field, 1 per calendar slot (0 - off, 1 - on). The in-band flow control can use reserved fields or management channel (again, not used for C2C applications). Also, the flow control can include an out-of-band mechanism with pins, aligned to FlexE frame boundaries. The out-of-band flow control can apply to normal FlexE channelization scheme or the new logical channel one.

## Chip-to-Chip (C2C) interface utilizing Flexible Ethernet (FlexE)

In an embodiment, a Chip-to-Chip (C2C) interface utilizing Flexible Ethernet (FlexE) includes circuitry configured to provide the FlexE over backplane/fabric links between two devices, wherein the circuitry includes flow control and channelization for the FlexE. Each of the two devices can include any of a Network Processor (NPU), a Fabric Interface Card (FIC), a framer, and a mapper, i.e., the framer/mapper 204 or the NPU/FIC 202. A rate of the FlexE group can be increased to support additional information for the flow control and the channelization. The circuitry, for the channelization, can be configured to provide a logical channel that shares one of a single calendar slot and a group of calendar slots. The logical channel can change on a frame boundary. Information associated with the logical channel can be communicated in FlexE overhead. The information can be provided in one of a reserved area of the FlexE overhead and a management channel of the FlexE overhead, wherein the management channel is not used in a C2C application. A calendar area of the FlexE overhead can be repurposed for the information. The information can be provided utilizing one of a defined Operational code and a data block immediately following a frame header. The circuitry, for the flow control, can be configured to provide flow control in a FlexE shim. The the flow control can include an in-band mechanism for XON-XOFF flow control per calendar slot, and wherein the flow control is communicated in one of a reserved area of FlexE overhead and a management channel of the FlexE overhead, wherein the management channel is not used in a C2C application.

## PTP

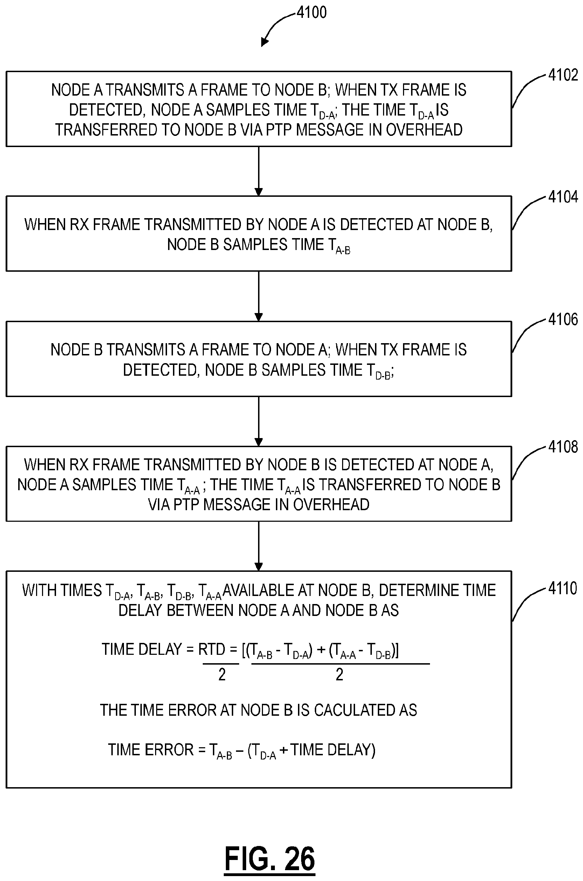

FIG. 26 is a flowchart of a time transfer process 4100 using a stream of Ethernet blocks. The time transfer process 4100 uses a fixed (multi)frame boundary or an overhead toggling/counting bit as a reference for timestamp generation. The reference must be reliably detected (post-Forward Error Correction (FEC) processing) at given intervals. The time of detection becomes the time reference point or "significant instant" for the time transfer process 4100. The time reference point is post adaptation/mapping processing in the transmit (TX) direction and pre adaptation/mapping in the receive (RX) direction to minimize the uncertainty and jitter introduced by such blocks.

The time reference point used to indicate an Arrival Time is $T_A$ using RX frame boundaries and Departure Time $T_D$ using TX frame boundaries. The PMD/PHY layers in the stack are Constant Bit Rate (CBR) and not significant contributors to CTE. The period for time reference point in the time transfer process 4100 must allow for the transfer of the timing information via a sync message channel, which can either use a reserved FlexE OH field or share the existing management channel.

The time transfer process 4100 is described between two nodes - node A and node B utilizing a stream of blocks. The node A transmits a frame towards node B; when the transmit frame is detected, node A samples time $T_{D-A}$; the time $T_{D-A}$ is transferred to the node B via a PTP message in the overhead (step 4102). When the receive frame transmitted by node A is detected at node B, node B samples time $T_{A-B}$ (step 4104). Node B transmits a frame to node A; when the transmit frame is detected, node B samples time $T_{D-B}$ (step 4106). When the receive frame transmitted by node B is detected at node A; node A samples time $T_{A-A}$; the time $T_{A-A}$ is transferred to node B via a PTP message in the overhead (step 4108). With times $T_{D-A}$, $T_{A-B}$, $T_{D-B}$, $T_{A-A}$ available at node B, the time delay between node A and node B is determined as:

$$TIME\_DELAY = \frac{RTD}{2} = \frac{[(T_{A-B} - T_{D-A}) + (T_{A-A} - T_{D-B})]}{2}$$

Where RTD is the Round Trip Delay which is $[(T_{A-B} - T_{D-A}) + (T_{A-A} - T_{D-B})]$. The time error at node B is calculated as:

$$TIME\_ERROR = T_{A-B} - (T_{D-A} + TIME\_DELAY) \quad \text{(step 110)}.$$

Control algorithms and fixed known offsets can then be employed to minimize the time error.

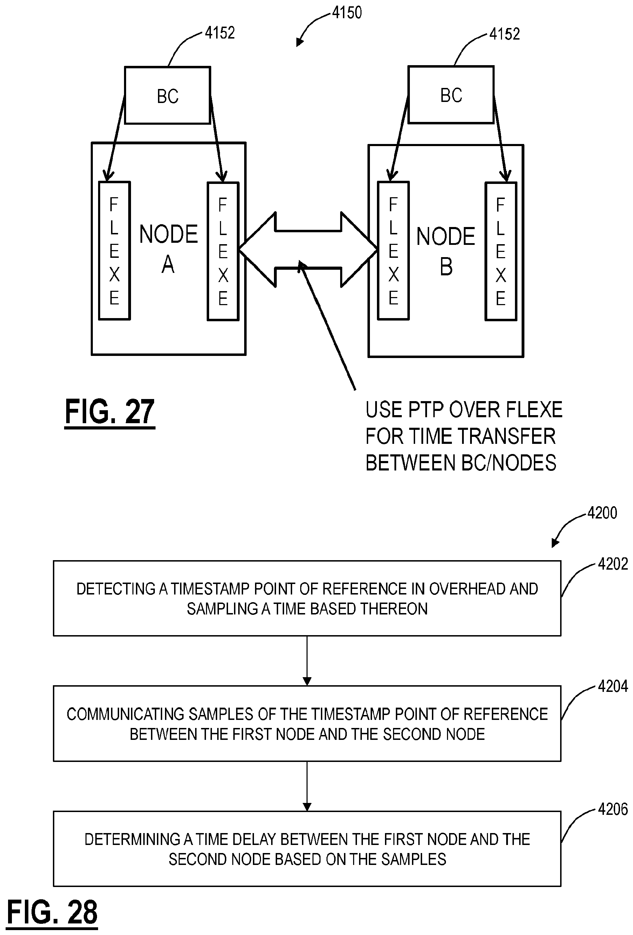

FIG. 27 is a network diagram of a network 4150 with node A and node B connected to one another via a stream of Ethernet blocks. Specifically, the time transfer process 4100 uses interfaces between boundary clocks 4152 and does not rely on clients. The boundary clock 4152 is an IEEE 1588 component that allows the synchronization of IEEE 1588 clocks across subnets defined by a router or other devices that blocks the transmission of all IEEE 1588 messages. The boundary clock 4152 serves to eliminate the large fluctuations in communication latency typically generated by routers and similar devices.

## FlexE Sync Messages

In the time transfer process 4100, messages are provided between nodes to relay the various time measurements. For example, in the time transfer process 4100, the node A transfers the times $T_{D-A}$, $T_{A-A}$ to the node B. In an embodiment, the time transfer process 4100 can use the reserved area in the overhead multiframe (shim overhead) for inserting these PTP messages. In another embodiment, the time transfer process 4100 can use the existing management channel for inserting these PTP messages. The management channel can use a Generic Framing Procedure - Framed (GFP-F)/High-Level Data Link Control (HDLC)/Ethernet encapsulation with some type of packet differentiation (i.e. GFP UPI).

**Time transfer method**

FIG. 28 is a flowchart of a time transfer method 4200 between a first node and a second node. The time transfer method 4200 includes detecting a timestamp point of reference in overhead and sampling a time based thereon (step 4202); communicating samples of the timestamp point of reference between the first node and the second node (step 4204); and determining a time delay between the first node and the second node based on the samples (step 4206). The timestamp point of reference can be one of a frame and multiframe boundary associated with frame TDM structure. The timestamp point of reference can be a toggling bit in the overhead. The timestamp point of reference is determined post adaptation/mapping processing in a transmit direction and pre adaptation/mapping in a receive direction. Step 4204 can be performed using a reserved area in the overhead or using a management channel in the overhead. The samples can include $T_{A-B}$ which is an arrival time in a receive direction at the second node, $T_{D-A}$ which is a departure time in a transmit direction at the first node, $T_{A-A}$ which is an arrival time in a receive direction at the first node, and $T_{D-B}$ which is a departure time in a transmit direction at the second node. A Round Trip Delay is $[(T_{A-B} - T_{D-A}) + (T_{A-A} - T_{D-B})]$, time delay TD is RTD/2, and a time error at the second node relative to the first node is $T_{A-B} - (T_{D-A} + TD)$.

**AES Encryption**

AES is a specification for the encryption of electronic data established by the U.S. National Institute of Standards and Technology (NIST). AES is based on the Rijndael cipher which is a family of ciphers with different key and block sizes. For AES, NIST selected three members of the Rijndael family, each with a block size of 128 bits, but three different key lengths: 128, 192 and 256 bits. AES is a symmetric-key algorithm, meaning the same key is used for both encrypting and decrypting the data. AES is based on a design principle known as a substitution-permutation network, combination of both substitution and permutation, and is fast in both software and hardware. Unlike its predecessor DES, AES does not use a Feistel network. AES operates on a $4 \times 4$ column-major order matrix of bytes, termed the state, although some versions of Rijndael have a larger block size and have additional columns in the state. Most AES calculations are done in a special finite field.

**GCM Encryption**

Galois/Counter Mode (GCM) is a mode of operation for symmetric key cryptographic block ciphers that has been widely adopted because of its efficiency and performance. GCM throughput rates for state of the art, high speed communication channels can be achieved with reasonable hardware resources. The operation is an authenticated encryption algorithm designed to provide both data authenticity (integrity) and confidentiality. GCM is defined for block ciphers with a block size of 128 bits. Galois Message Authentication Code (GMAC) is an authentication-only variant of the GCM which can be used as an incremental message authentication code. Both GCM and GMAC can accept initialization vectors of arbitrary length. Different block cipher modes of operation can have significantly different performance and efficiency characteristics, even when used with the same block cipher. GCM can take full advantage of parallel processing and implementing GCM can make efficient use of an instruction pipeline or a hardware pipeline. In contrast, the cipher block chaining (CBC) mode of operation incurs significant pipeline stalls that hamper its efficiency and performance.

**Encryption**

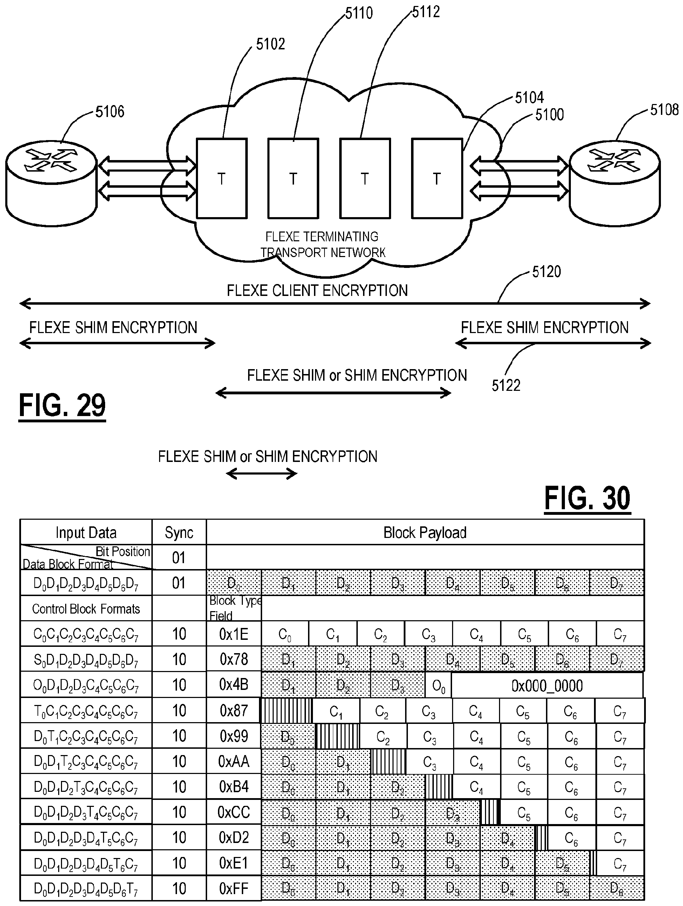

FIG. 29 is a network diagram of a terminating transport network 5100 with network elements 5102, 5104 coupled to switches/routers 5106, 5108 and with intermediate, transparent network elements 5110, 5112 between the network elements 5102, 5104, with encryption on a stream of 257b blocks. In an embodiment, a FlexE client can be transported as a service (or private line service) in a provider network (i.e., the transport network 5100). The switches/routers 5106, 5108 can be referred to as subscriber equipment where a service originates or terminates, i.e., a maintenance entity. The transport network 5100 can include a plurality of network elements in addition to the network elements 5102, 5104. The various network elements in the transport network 5100 are configured to perform transport/switching. In FlexE, the transport/switching can be ODUk/flex and/or FlexE client based switching.

The transport network 5100 can include FlexE client encryption 5120, covering the entire path, and/or FlexE shim encryption 5122, covering the section between the router to transport segment. Further, FlexE client encryption can cover the path from on-ramp transport node to off-ramp transport node. Additionally, shim encryption can also cover the segments between transport nodes as well as cover the path layer between on-ramp and off-ramp transport nodes. In either of the encryption methods 5120, 5122, encryption is applied at Layer 1, does not affect the packet rate and can be used with future interfaces, without requiring need for OTN wrapping. The encryptions 5120, 5122 are also lower complexity, resulting in lower logic, lower power and lower cost for the overall solution. The client encryption 5120 is applied at the FlexE client (i.e., path layer) between the switches/routers 5106, 5108 and across the transport network 5100. The shim encryption 5122 is applied at the FlexE shim (i.e., section/segment) between the switch/router 106 and the network element 102 and between the switch/router 5108 and the network element 5104. The FlexE shim encryption can be applied at the FlexE shim between the on-ramp network element (5102, 5104) and the off-ramp network element (5104, 5102). The FlexE shim encryption can be applied at the FlexE shim between individual transport network elements 5102, 5104, 5110, 5112. The FlexE client encryption can be applied at the FlexE client between the on-ramp network element 5102, 5104 and the off-ramp network element 5104, 5102. The FlexE client encryption can be applied at the FlexE client between individual transport network elements 5102, 5104, 5110, 5112.

The FlexE shim can reside between the RS layer and the PMD. For the FlexE shim encryption 5122, encryption is provided at the FlexE shim, i.e., on the whole shim. 256b/257b bit streams for all clients are mapped into the shim and the shim encryption 5122 can be applied across the entire shim, i.e., all of the clients. The shim encryption 5122 does not benefit to client switching as it is applied hop by hop, but it is less complex (than the client encryption 5120) and can be applied without knowledge of the client mappings (i.e., in the transport equipment or the PHYs). The FlexE shim encryption 5122 can be applied at the bottom of the FlexE shim layer. The FlexE shim encryption 5122 can reuse portions of the FlexE frame overhead for key exchange, authentication, GCM tags, etc. In an embodiment, an encryption messaging channel for key exchange, authentication, GCM tags, etc. can utilize reserved and/or undefined locations in the FlexE frame/shim overhead. Also, the encryption messaging channel can be performed through the FlexE management channel.

The client encryption 5120 can be applied on individual clients, and can be performed at a top of the shim. The FlexE client encryption 5120 benefits client switching, and can be mapped into the shim or can be a standard client going to standard PCS/PHY. Also, the client encryption 5120 can be utilized with the shim encryption 5122. For communications, the client encryption 5120 can utilize a portion of the client stream (e.g., 1/16k) with a special O code (e.g., 0x9) with 24-bit data fields for an encryption messaging channel.

FIG. 30 is a table of 256b/257b block formats. For the client encryption 120 at 256b/257b, the client encryption 120 does not encrypt the 256b/257b header as this still needs to go through standard Ethernet PHYs and is used for alignment. For the client encryption 5120, 01 data blocks (Dx fields) can be all encrypted and 10 control block need special attention. In an embodiment, only data blocks are encrypted which allows idles and rate adjustments, allows for LF/RF, and easily maps into ODUflex(IMP). In another embodiment, data and some control blocks are encrypted to minimize the visibility of the Start of Packet (SOP)/End of Packet (EOP) (link chatter).

GCM can be used in combination with AES to provide authentication of the encrypted message by adding a tag (hash). As per NIST Special Publication 800-38D, November 2007, "Recommendation for Block Cipher Modes of Operation: Galois/Counter Mode (GCM) and GMAC," up to 64GB of data can be covered with a single tag of 96, 104, 112, 120, or 128 bits. There is less than 50ppm to add the GCM tag. As much of that as possible can be used to minimize latency and buffer requirements. The GCM tag is either in the shim layer encryption messaging channel (for the shim encryption 5122) or directly in-band with the 0x9 O-code message channel when encrypting the client directly (for the FlexE client encryption 5120).

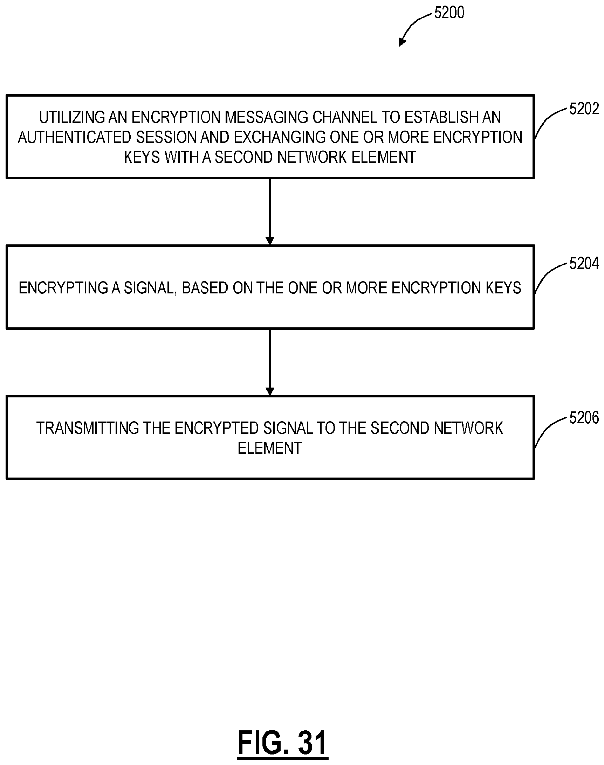

FIG. 31 is a flowchart of a process 5200 for Physical Coding Sublayer (PCS) encryption implemented by a first network element communicatively coupled to a second network element. The process 5200 includes utilizing an encryption messaging channel to establish an authenticated session and exchanging one or more encryption keys with a second network element (step 5202); encrypting a signal, based on the one or more encryption keys (step 5204); and transmitting the encrypted signal to the second network element (step 5206). The encryption can be applied to a 256b/257b bit stream associated with a signal at one or more of a client layer and a shim layer. The encryption messaging channel can be utilized for key exchange, authentication, and tags with the second network element. The signal can include a shim including a plurality of clients. The signal can include one of a client and a generic Ethernet PCS stream onto standard Ethernet PHY. The encryption messaging channel can utilize one or more of reserved bytes in overhead and a management channel. The encryption messaging channel can utilize a client or PCS stream with a designed Operational code. The encryption can be applied to a 256b/257b bit stream with data blocks encrypted as well as some control blocks encrypted. The encryption can utilize one or more of Advanced Encryption Standard (AES) and Galois/Counter Mode (GCM).

It will be appreciated that some embodiments described herein may include one or more generic or specialized processors ("one or more processors") such as microprocessors; Central Processing Units (CPUs); Digital Signal Processors (DSPs): customized processors such as Network Processors (NPs) or Network Processing Units (NPUs), Graphics Processing Units (GPUs), or the like; Field Programmable Gate Arrays (FPGAs); and the like along with unique stored program instructions (including both software and firmware) for control thereof to implement, in conjunction with certain non-processor circuits, some, most, or all of the functions of the methods and/or systems described herein. Alternatively, some or all functions may be implemented by a state machine that has no stored program instructions, or in one or more Application Specific Integrated Circuits (ASICs), in which each function or some combinations of certain of the functions are implemented as custom logic or circuitry. Of course, a combination of the aforementioned approaches may be used. For some of the embodiments described herein, a corresponding device in hardware and optionally with software, firmware, and a combination thereof can be referred to as "circuitry configured or adapted to," "logic configured or adapted to," etc. perform a set of operations, steps, methods, processes, algorithms, functions, techniques, etc. on digital and/or analog signals as described herein for the various embodiments.

Moreover, some embodiments may include a non-transitory computer-readable storage medium having computer readable code stored thereon for programming a computer, server, appliance, device, processor, circuit, etc. each of which may include a processor to perform functions as described and claimed herein. Examples of such computer-readable storage mediums include, but are not limited to, a hard disk, an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory), Flash memory, and the like. When stored in the non-transitory computer-readable medium, software can include instructions executable by a processor or device (e.g., any type of programmable circuitry or logic) that, in response to such execution, cause a processor or the device to perform a set of operations, steps, methods, processes, algorithms, functions, techniques, etc. as described herein for the various embodiments.

Although the present disclosure has been illustrated and described herein with reference to preferred embodiments and specific examples thereof, it will be readily apparent to those of ordinary skill in the art that other embodiments and examples may perform similar functions and/or achieve like results.

**Claims**

1. A method comprising:

   receiving a first Ethernet frame (10) having 66-bit (66b) blocks including 66b overhead blocks (12) and 66b data blocks (14);
   buffering received 66b overhead blocks (12) and 66b data blocks (14);
   mapping four 66b overhead blocks (12) from the buffering into a 257-bit (257b) overhead block (52) and a sequence of four 66b data blocks (14) from the buffering into a 257b data block (54), wherein the mapping includes accumulating four 66b blocks of a same kind from the buffering for mapping into a 257b block, wherein the same kind is one of overhead and a particular calendar slot n where n = 0 - 19; wherein each overhead block (12, 52) is located in a same sequence in the 66b blocks and the 257b blocks, and
   **characterized by** transmitting a second Ethernet frame (50) that is one of Flexible Ethernet, FlexE, and Metro Transport Networking, MTN having 257b blocks from the mapping,
   wherein both the first Ethernet frame (10) and the second Ethernet frame (50) include 20 calendar slots for Ethernet client data, wherein each calendar slot in the first Ethernet frame (10) is a 66b block, and wherein the second Ethernet frame (50) has four calendar slots of a same kind in each 257b data block, wherein each overhead block (12, 52) is used as a marker for framing.

2. The method as claimed in claim 1, further comprising:

   receiving in a demapping circuit a third Ethernet frame having 257b blocks including 257b overhead blocks (52) and 257b data blocks (54);
   wherein a second buffer (90) is connected to the demapping circuit, and demapping the 257b overhead blocks (52) into four 66b overhead blocks (12) that are stored in the second buffer (90) and demapping the 257b data blocks (54) into four 66b data blocks (14) that are stored in the second buffer (90); and
   transmitting a fourth Ethernet frame (10) having 66b blocks from the second buffer (90).

3. The method as claimed in claim 2, wherein the sequence includes 1023 repetitions of the 20 four accumulated calendar slots between overhead blocks.

4. The method as claimed in claim 3, wherein both the first Ethernet frame (10) and the second Ethernet frame (50) utilize a same calendar slot distribution scheme.

5. The method as claimed in any one of claims 1-4, wherein the first Ethernet frame (10) utilizes a multiframe approach where each 66b overhead block is one of eight blocks, and wherein the second Ethernet frame (50) maintains the multiframe approach where two 257b overhead blocks support the eight blocks.

6. The method as claimed in any one of claims 1-5, wherein the 66b overhead blocks (12) and the 66b data blocks (14) are separately buffered.

7. The method as claimed in any one of claims 1-6, wherein a pointer in the 66b overhead blocks is utilized to coordinate addition or removal of Ethernet clients in the 66b data blocks (14).

8. A circuit (80) configured to implement the method as claimed in any one of claims 1-7.

**Patentansprüche**

1. Verfahren, umfassend:

Empfangen eines ersten Ethernet-Frames (10) aufweisend 66-Bit-Blöcke (66b) einschließlich 66b-Overhead-Blöcken (12) und 66b-Datenblöcken (14);
Pufferung empfangener 66b-Overhead-Blöcke (12) und 66b-Datenblöcke (14);
Mapping von vier 66b-Overhead-Blöcken (12) aus der Pufferung in einen 257-Bit-(257b)-Overhead-Block (52) und einer Folge von vier 66b-Datenblöcken (14) aus der Pufferung in einen 257b-Datenblock (54), wobei das Mapping das Akkumulieren von vier 66b-Blöcken derselben Art aus der Pufferung zum Mapping in einen 257b-Block einschließt, wobei die gleiche Art eine von Overhead und einem bestimmten Kalender-Slot n ist, wobei n = 0 -19 ist; wobei jeder Overhead-Block (12, 52) in der gleichen Reihenfolge in den 66b-Blöcken und den 257b-Blöcken angeordnet ist, und
**Gekennzeichnet durch** Übertragen eines zweiten Ethernet-Frames (50), der eines ist von Flexible Ethernet, FlexE und Metro Transport Networking, MTN, aufweisend 257b-Blöcke aus dem Mapping,
wobei sowohl der erste Ethernet-Frame (10) als auch der zweite Ethernet-Frame (50) 20 Kalender-Slot für Ethernet-Client-Daten einschließen, wobei jeder Kalender-Slot in dem ersten Ethernet-Frame (10) ein 66b-Block ist, und wobei der zweite Ethernet-Frame (50) vier Kalender-Slot derselben Art in jedem 257b-Datenblock aufweist, wobei jeder Overhead-Block (12, 52) als ein Marker für Framing verwendet wird.

2. Verfahren nach Anspruch 1, weiter umfassend:

Empfangen, in einer Demapping-Schaltung, eines dritten Ethernet-Frames aufweisend 257b Blöcke einschließlich 257b Overhead-Blöcken (52) und 257b Datenblöcken (54);
wobei ein zweiter Puffer (90) mit der Demapping-Schaltung verbunden ist, und Demapping der 257b-Overhead-Blöcke (52) in vier 66b-Overhead-Blöcke (12), die in dem zweiten Puffer (90) gespeichert sind, und Demapping der 257b-Datenblöcke (54) in vier 66b-Datenblöcke (14), die in dem zweiten Puffer (90) gespeichert sind; und
Übertragen eines vierten Ethernet-Frames (10) aufweisend 66b Blöcke aus dem zweiten Puffer (90).

3. Verfahren nach Anspruch 2, wobei die Sequenz 1023 Wiederholungen der 20 vier akkumulierten Kalender-Slot zwischen Overhead-Blöcken einschließt.

4. Verfahren nach Anspruch 3, wobei sowohl der erste Ethernet-Frame (10) als auch der zweite Ethernet-Frame (50) das gleiche Verteilungsschema für Kalender-Slots verwenden.

5. Verfahren nach einem der Ansprüche 1-4, wobei der erste Ethernet-Frame (10) einen Multiframe-Ansatz verwendet, bei dem jeder 66b-Overhead-Block einer von acht Blöcken ist, und wobei der zweite Ethernet-Frame (50) den Multiframe-Ansatz beibehält, bei dem zwei 257b-Overhead-Blöcke die acht Blöcke unterstützen.

6. Verfahren nach einem der Ansprüche 1-5, wobei
die 66b-Overhead-Blöcke (12) und die 66b-Datenblöcke (14) getrennt gepuffert werden.

7. Verfahren nach einem der Ansprüche 1-6, wobei ein Zeiger in den 66b-Overhead-Blöcken verwendet wird, um das Hinzufügen oder Entfernen von Ethernet-Clients in den 66b-Datenblöcken (14) zu koordinieren.

8. Schaltung (80), die konfiguriert ist, um das Verfahren nach einem der Ansprüche 1-7 durchzuführen.

**Revendications**

1. Procédé comprenant :

   la réception d'un premier cadre Ethernet (10) présentant des blocs de 66 bits (66b) incluant des blocs de surdébit 66b (12) et des blocs de données 66b (14) ;
   la mise en mémoire tampon de blocs de surdébit 66b (12) et de blocs de données 66b (14) reçus ;
   le mappage de quatre blocs de surdébit 66b (12) à partir de la mise en mémoire tampon dans un bloc de surdébit de 257 bits (257b) (52) et une séquence de quatre blocs de données 66b (14) à partir de la mise en mémoire tampon dans un bloc de données 257b (54), dans lequel le mappage inclut l'accumulation de quatre blocs 66b d'un même type à partir de la mise en mémoire tampon pour le mappage dans un bloc 257b, dans lequel le même type est un parmi le surdébit et une fente calendaire particulière n où n = 0-19 ; dans lequel chaque bloc de surdébit (12, 52) est situé dans une même séquence dans les blocs 66b et les blocs 257b, et
   **caractérisé par** la transmission d'un second cadre Ethernet (50) qui est un parmi Flexible Ethernet, FlexE, et Métro Transport Networking, MTN présentant des blocs 257b à partir du mappage,
   dans lequel à la fois le premier cadre Ethernet (10) et le deuxième cadre Ethernet (50) incluent 20 fentes calendaires pour des données client Ethernet, dans lequel chaque fente calendaire dans le premier cadre Ethernet (10) est un bloc 66b, et dans lequel le second cadre Ethernet (50) présente quatre fentes calendaires d'un même type dans chaque bloc de données 257b, dans lequel chaque bloc de surdébit (12, 52) est utilisé comme un marqueur pour le cadrage.

2. Procédé selon la revendication 1, comprenant en outre :

   la réception dans un circuit de démappage d'un troisième cadre Ethernet présentant des blocs 257b incluant des blocs de surdébit 257b (52) et des blocs de données 257b (54) ;
   dans lequel une seconde mémoire tampon (90) est reliée au circuit de démappage, et le démappage des blocs de surdébit 257b (52) dans quatre blocs de surdébit 66b (12) qui sont stockés dans la seconde mémoire tampon (90) et le démappage des blocs de données 257b (54) dans quatre blocs de données 66b (14) qui sont stockés dans la seconde mémoire tampon (90) ; et
   la transmission d'un quatrième cadre Ethernet (10) présentant des blocs 66b à partir de la seconde mémoire tampon (90).

3. Procédé selon la revendication 2, dans lequel la séquence inclut 1023 répétitions des quatre fentes calendaires accumulées 20 entre des blocs de surdébit.

4. Procédé selon la revendication 3, dans lequel à la fois le premier cadre Ethernet (10) et le deuxième cadre Ethernet (50) utilisent un même schéma de distribution de fente calendaire.

5. Procédé selon l'une quelconque des revendications 1-4, dans lequel le premier cadre Ethernet (10) utilise une approche multicadre où chaque bloc de surdébit 66b est un des huit blocs, et dans lequel le deuxième cadre Ethernet (50) maintient l'approche multicadre où deux blocs de surdébit 257b supportent les huit blocs.

6. Procédé selon l'une quelconque des revendications 1-5, dans lequel
   les blocs de surdébit 66b (12) et les blocs de données 66b (14) sont mis en mémoire tampon séparément.

7. Procédé selon l'une quelconque des revendications 1-6, dans lequel un pointeur dans les blocs de surdébit 66b est utilisé pour coordonner l'ajout ou le retrait de clients Ethernet dans les blocs de données 66b (14).

8. Circuit (80) configuré pour mettre en oeuvre le procédé selon l'une quelconque des revendications 1-7.

FIG. 1

EP 3 909 209 B1

SH  0 1 2 3 4 5 6 7 8 9 10 11 12 13 14 15 16 17 18 19 20 21 22 23 24 25 26 27 28 29 30 31 32 33 34 35 36 37 38 39 40 41 42 43 44 45 46 47 48 49 50 51 52 53 54 55 56 57 58 59 60 61 62 63

| | | | | | | | |
|---|---|---|---|---|---|---|---|
| 1 | 1 0 | 0x4B | C OMF RPF SF | FlexE Group Number | 0x5 | 0x000_0000 | |
| 2 | 0 1 | C | FlexE Map / FlexE Instance Num. | Reserved | | | |
| 3 | 0 1 | C | Client Calendar A | Client Calendar B | CR CA | Reserved | CRC-16 |
| 4 | s s | | Management Channel – Section (two 66B blocks) | | | | |
| 5 | s s | | | | | | |
| 6 | s s | | Management Channel – Shim to Shim or Synchronization Messaging Channel | | | | |
| 7 | s s | | Management Channel – Shim to Shim (two or three 66B blocks) | | | | |
| 8 | s s | | | | | | |

16 FlexE Overhead Frames

| 0 | FlexE Map (0-7) | Client carried Calendar A slot 0 | Client carried Calendar B slot 0 |
| 0 | FlexE Map (8-15) | Client carried Calendar A slot 1 | Client carried Calendar B slot 1 |
| | • • • | • • • | • • • |
| 0 | FlexE Map (120-127) | Client carried Calendar A slot 15 | Client carried Calendar B slot 15 |
| 1 | FlexE Map (128-135) | Client carried Calendar A slot 16 | Client carried Calendar B slot 16 |

16 FlexE Overhead Frames

| | • • • | • • • | • • • |
| 1 | FlexE Map (151-159) | Client carried Calendar A slot 19 | Client carried Calendar B slot 19 |
| 1 | FlexE Map (160-167) | Reserved | Reserved |
| | • • • | • • • | • • • |
| 1 | FlexE Map (248-255) | Reserved | Reserved |

C = Calendar configuration in use (see 7.3.2)
OMF = Overhead Multiframe Indicator (see 7.3.1)
C = Calendar configuration in use (see 7.3.2)
RPF = Remote PHY Fault (see 7.3.8)
SC = Synchronization Configuration – see 7.3.5
CR = Calendar Switch Request (see 7.3.4)
CA = Calendar Switch Acknowledge (see 7.3.4)
s s = Valid sync header bit (01 or 10)

FlexE Map = Control of which 100G FlexE instances are members of this group (see 7.3.3)
FlexE Instance Number = identity of this 100G FlexE instance within the group (see 7.3.3)
Flex Group Number – see 7.3.6
Client (being programmed) – see 7.3.4
Reserved – See 7.3.7
Management Channels – see 7.3.5
CRC-16 – See 7.3.8
Synchronization Messaging Channel – See 7.3.5

## FIG. 2

| 1 | DB1(64) | DB2(64) | DB3(64) | DB4(64) |
|---|---|---|---|---|

26 ⟋    20 ↙

**FIG. 3**

| 0 | 0010 | F1(4) | CB1(56) | F2(4) | S2(4) | CB2(56) | DB3(64) | F4(4) | S4(4) | CB4(56) |
|---|---|---|---|---|---|---|---|---|---|---|

26 ⟋    22 ↙    28 ⟋

**FIG. 4**

SAME REPETITION
EXCEPT WITH 257b
BLOCKS

1023 REPETITIONS OF CALENDAR BETWEEN FLEXE OVERHEAD BLOCKS

FLEXE OH IS A 257b
BLOCK WITH FOUR
FLEXE OVERHEAD 66b
BLOCKS

EACH BLOCK IS a 257b BLOCK WITH
FOUR 66b CALENDAR SLOTS, e.g.,
0000, 1111, 2222, 3333, …, 19191919

**FIG. 5**

FIG. 6

EP 3 909 209 B1

FIG. 7

FLEXE FRAME 10 — 84 BUFFER — MAPPING CIRCUITRY 86 — FLEXE FRAME 50

— 80

FLEXE FRAME 50 — DEMAPPING CIRCUITRY 88 — BUFFER 90 — FLEXE FRAME 10

— 82

## FIG. 8

| 01 | DB1(64) | 01 | DB1(64) | 01 | DB1(64) | 01 | DB1(64) |

| 1 | DB1(64) | DB1(64) | DB1(64) | DB1(64) |

**FIG. 9**

**FIG. 10**

EP 3 909 209 B1

┌─ P1

| IN ELECTRICAL CIRCUITRY, RECEIVING A FLEXIBLE ETHERNET (FLEXE) FRAME HAVING 66B BLOCKS INCLUDING 66B OVERHEAD BLOCKS AND 66B DATA BLOCKS | — S1 |

| BUFFERING RECEIVED 66B OVERHEAD BLOCKS AND 66B DATA BLOCKS | — S2 |

| MAPPING FOUR X 66B OVERHEAD BLOCKS FROM THE BUFFERING INTO A 257B OVERHEAD BLOCK AND A SEQUENCE OF FOUR X 66B DATA BLOCKS FROM THE BUFFERING INTO A 257B DATA BLOCK | — S3 |

| TRANSMITTING A FLEXE FRAME HAVING 257B BLOCKS BASED ON THE MAPPING STEPS | — S4 |

## FIG. 11

┌─ P2

| ASSEMBLING OVERHEAD INTO A FIRST 257B BLOCK OF A FLEXIBLE ETHERNET (FLEXE) FRAME, WHEREIN THE OVERHEAD INCLUDES ALIGNMENT DATA, CALENDAR INFORMATION, AND A MANAGEMENT CHANNEL | — S11 |

| PROVIDING A PLURALITY OF 257B DATA BLOCKS ACCORDING TO A CALENDAR AND SUBSEQUENT TO THE FIRST 257B BLOCK WITH THE OVERHEAD | — S12 |

| TRANSMITTING THE FLEXE FRAME HAVE THE FIRST 257B BLOCK AND THE PLURALITY OF 257B DATA BLOCKS | — S13 |

## FIG. 12

- N x clients
- M x groups (standard
PMD interfaces)
- i.e. 100GE

- Terminate shim OH
- Get access to 257b
blocks and slots
- Function likely in a
framer/mapper or switch
device

- Move around 257b
blocks to appropriate
fabric link
- Function likely in FIC

- Pack 257b blocks/slots
into new FlexE shim
(container) to go to
fabric
- Links can again use
standard groups/PMDs
i.e. 100GE

- Switch on a 257b
block boundary
- Completely agnostic
to content or
grouping of 257b
blocks

FIG. 13

At this point, aware of grouping. Could do
idle rate adaptation into new frequency
domain (analogy to pointer processing in
SDH)

If all synchronous, can
perform phase alignment to
new system ref (FlexE
frame)

Perform phase adjustment
to account for interface
skew

FIG. 14

31

**FIG. 15**

- N x clients
- M x groups (standard PMD interfaces)
- i.e. 100GE
- Terminate shim OH
- Get access to 64b66b blocks and slots

- Adapt (map/demap) 64b66b to STS-n
- Need to keep 64b66b intact

- Use standard TFI5 links (STS-48/60)

- Legacy STS switch

**FIG. 16**

420

406

410

- 402

416  412  408  414

400

404

418

line card 1

S

OPF
SARing and Timing
Transfer

fabric A

Ports are
async to
system
timing.

Phase Aligned Clock

OPF
SARing and Timing
Transfer

S

line card n

fabric B

- N x clients
- M x groups (standard
  PMD interfaces)
- i.e. 100GE
- Terminate shim OH
- Get access to 64b66b
  blocks and slots

• Use a phase aligned clock
  to deskew FlexE channels
  passing across the fabric as
  unique flows.
• The phase aligned clock
  allows time stamping of
  each cell thereby enabling
  deskewing at the egress.

• SAR: Adapt 64b66b
  TDM streams using
  OPF, Need to keep
  64b66b intact
• Timing Transfer:
  Required for Async
  port timing

- Use packet switching
  over cell switched links.

- Cell switch

## FIG. 17

420

406

410

- 402

416  412  408  414

400

404

418

line card 1

S

OPF
SARing

fabric A

Ports are
synchronous
to system
timing.

Phase Aligned Clock

OPF
SARing

S

line card n

fabric B

- N x clients
- M x groups (standard
  PMD interfaces)
- i.e. 100GE
- Terminate shim OH
- Get access to 64b66b
  blocks and slots

• Use a phase aligned clock
  to deskew FlexE channels
  passing across the fabric as
  unique flows.
• The phase aligned clock
  allows time stamping of
  each cell thereby enabling
  deskewing at the egress.

• SAR: Adapt 64b66b
  TDM streams using
  OPF, Need to keep
  64b66b intact
• Timing Transfer: Not
  Required for Synch
  port timing

- Use packet switching
  over cell switched links.

- Cell switch

## FIG. 18

FlexO UNI/IrDI/E-NNI interfaces

1106

FlexO

1100

1102

T

TRANSPORT
NETWORK

T

1104

1108

ODUflex
SERVICE
● ← m x OTUCn ← ● ← ODUflex
Transport/switching
● → m x OTUCn → ● ODUflex
SERVICE ← ●

service originating in subscriber equipment
(maintenance entity)

provider's network
multi-nodes of OTN or FlexE switching

service terminating in
subscriber equipment
(maintenance entity)

1106

FlexE

1102

T

TRANSPORT
NETWORK

T

1100

1104

1108

FlexE
Client
SERVICE
● ← m x FlexE
groups
← ● ← ODUflex/FlexE
Transport/switching
← ● m x FlexE
groups
→ ● → FlexE
Client
SERVICE → ●

FlexE UNI/IrDI/E-NNI interfaces

## FIG. 19

monitoring points

TRANSPORT
NETWORK

FlexE client
SERVICE

mxFlexE
groups

FlexE
Transport/switching

mxFlexE
groups

FlexE client
SERVICE

## FIG. 20

RECEIVING A CLIENT

1202

AT LEAST ONE OF MONITORING AND UPDATING ONE OR
MORE OPERATIONS, ADMINISTRATION, AND MAINTENANCE
(OAM) FIELDS IN OVERHEAD, WHEREIN THE OAM FIELDS
COVER A SINGLE CLIENT PATH FOR THE CLIENT

1204

## FIG. 21

**FIG. 22**

FIG. 23

EP 3 909 209 B1

This could be an Ethernet client port
or the client side of a FlexE shim

This could be a packet I/F such as
Interlaken

CBR MAPPING EXAMPLE

ETHERNET SIGNAL

MAC FRAMES

| ETHERNET | ETHERNET PDU | OTHER PDU-BASED PROTOCOLS (IP, MPLS) |
| | | LLC OR OTHER MAC CLIENT |
| | | MAC CONTROL (OPTIONAL) |
| | ETHERNET MAC/RS | |
| | ETHERNET PCS | |
| ADAPTATION TO ODUflex | | |
| B100G OPUflex | | |

ETH ADAPTATION FUNC

B100G OPUflex

MAC/RS/PCS

ETHERNET SIGNAL

ETH ADAPTATION FUNC

B100G OPUflex

# FIG. 24

—3200

—3202

—3204

| FlexE | NPU/FIC | FlexE | FRAMER/ MAPPER | OTN |

Historically would be Interlaken or fixed PMDs

## FIG. 25

4100

┌─────────────────────────────────────────────────────────────┐
│ NODE A TRANSMITS A FRAME TO NODE B; WHEN TX FRAME IS          │  4102
│ DETECTED, NODE A SAMPLES TIME $T_{D-A}$; THE TIME $T_{D-A}$ IS │
│ TRANSFERRED TO NODE B VIA PTP MESSAGE IN OVERHEAD             │
└─────────────────────────────────────────────────────────────┘

┌─────────────────────────────────────────────────────────────┐
│ WHEN RX FRAME TRANSMITTED BY NODE A IS DETECTED AT NODE B,    │  4104
│ NODE B SAMPLES TIME $T_{A-B}$                                 │
└─────────────────────────────────────────────────────────────┘

┌─────────────────────────────────────────────────────────────┐
│ NODE B TRANSMITS A FRAME TO NODE A; WHEN TX FRAME IS          │  4106
│ DETECTED, NODE B SAMPLES TIME $T_{D-B}$;                      │
└─────────────────────────────────────────────────────────────┘

┌─────────────────────────────────────────────────────────────┐
│ WHEN RX FRAME TRANSMITTED BY NODE B IS DETECTED AT NODE A,    │  4108
│ NODE A SAMPLES TIME $T_{A-A}$; THE TIME $T_{A-A}$ IS TRANSFERRED TO NODE B │
│ VIA PTP MESSAGE IN OVERHEAD                                   │
└─────────────────────────────────────────────────────────────┘

┌─────────────────────────────────────────────────────────────┐
│ WITH TIMES $T_{D-A}$, $T_{A-B}$, $T_{D-B}$, $T_{A-A}$ AVAILABLE AT NODE B, DETERMINE TIME │  4110
│ DELAY BETWEEN NODE A AND NODE B AS                           │
│                                                             │
│ $$\text{TIME DELAY} = \frac{RTD}{2} = \frac{[(T_{A-B} - T_{D-A}) + (T_{A-A} - T_{D-B})]}{2}$$ │
│                                                             │
│ THE TIME ERROR AT NODE B IS CACULATED AS                    │
│                                                             │
│ $$\text{TIME ERROR} = T_{A-B} - (T_{D-A} + \text{TIME DELAY})$$ │
└─────────────────────────────────────────────────────────────┘

## FIG. 26

## FIG. 27

USE PTP OVER FLEXE
FOR TIME TRANSFER
BETWEEN BC/NODES

DETECTING A TIMESTAMP POINT OF REFERENCE IN OVERHEAD AND
SAMPLING A TIME BASED THEREON

COMMUNICATING SAMPLES OF THE TIMESTAMP POINT OF REFERENCE
BETWEEN THE FIRST NODE AND THE SECOND NODE

DETERMINING A TIME DELAY BETWEEN THE FIRST NODE AND THE
SECOND NODE BASED ON THE SAMPLES

## FIG. 28

FLEXE CLIENT ENCRYPTION

FLEXE SHIM ENCRYPTION

FLEXE SHIM ENCRYPTION

FLEXE SHIM or SHIM ENCRYPTION

## FIG. 29

FLEXE SHIM or SHIM ENCRYPTION

## FIG. 30

| Input Data | Sync | Block Payload | | | | | | | | |
|---|---|---|---|---|---|---|---|---|---|---|
| Bit Position / Data Block Format | 01 | | | | | | | | | |
| $D_0D_1D_2D_3D_4D_5D_6D_7$ | 01 | $D_0$ | $D_1$ | $D_2$ | $D_3$ | $D_4$ | $D_5$ | $D_6$ | $D_7$ | |
| Control Block Formats | | Block Type Field | | | | | | | | |
| $C_0C_1C_2C_3C_4C_5C_6C_7$ | 10 | 0x1E | $C_0$ | $C_1$ | $C_2$ | $C_3$ | $C_4$ | $C_5$ | $C_6$ | $C_7$ |
| $S_0D_1D_2D_3D_4D_5D_6D_7$ | 10 | 0x78 | $D_1$ | $D_2$ | $D_3$ | $D_4$ | $D_5$ | $D_6$ | $D_7$ | |
| $O_0D_1D_2D_3C_4C_5C_6C_7$ | 10 | 0x4B | $D_1$ | $D_2$ | $D_3$ | $O_0$ | 0x000_0000 | | | |
| $T_0C_1C_2C_3C_4C_5C_6C_7$ | 10 | 0x87 | | $C_1$ | $C_2$ | $C_3$ | $C_4$ | $C_5$ | $C_6$ | $C_7$ |
| $D_0T_1C_2C_3C_4C_5C_6C_7$ | 10 | 0x99 | $D_0$ | | $C_2$ | $C_3$ | $C_4$ | $C_5$ | $C_6$ | $C_7$ |
| $D_0D_1T_2C_3C_4C_5C_6C_7$ | 10 | 0xAA | $D_0$ | $D_1$ | | $C_3$ | $C_4$ | $C_5$ | $C_6$ | $C_7$ |
| $D_0D_1D_2T_3C_4C_5C_6C_7$ | 10 | 0xB4 | $D_0$ | $D_1$ | $D_2$ | | $C_4$ | $C_5$ | $C_6$ | $C_7$ |
| $D_0D_1D_2D_3T_4C_5C_6C_7$ | 10 | 0xCC | $D_0$ | $D_1$ | $D_2$ | $D_3$ | | $C_5$ | $C_6$ | $C_7$ |
| $D_0D_1D_2D_3D_4T_5C_6C_7$ | 10 | 0xD2 | $D_0$ | $D_1$ | $D_2$ | $D_3$ | $D_4$ | | $C_6$ | $C_7$ |
| $D_0D_1D_2D_3D_4D_5T_6C_7$ | 10 | 0xE1 | $D_0$ | $D_1$ | $D_2$ | $D_3$ | $D_4$ | $D_5$ | | $C_7$ |
| $D_0D_1D_2D_3D_4D_5D_6T_7$ | 10 | 0xFF | $D_0$ | $D_1$ | $D_2$ | $D_3$ | $D_4$ | $D_5$ | $D_6$ | |

5200

UTILIZING AN ENCRYPTION MESSAGING CHANNEL TO ESTABLISH AN AUTHENTICATED SESSION AND EXCHANGING ONE OR MORE ENCRYPTION KEYS WITH A SECOND NETWORK ELEMENT — 5202

ENCRYPTING A SIGNAL, BASED ON THE ONE OR MORE ENCRYPTION KEYS — 5204

TRANSMITTING THE ENCRYPTED SIGNAL TO THE SECOND NETWORK ELEMENT — 5206

## FIG. 31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEAN-MICHEL CAIA.** Flex025n Multiplexing and Overhead structures for 25G/50G M-OTN interfaces; WD11-24. *ITU-T draft,* 28 May 2018, vol. 11 /15, 12/15, 1-16, https://www.itu.int/ifa/ifa/t/2017/sg15/exchange/wp3/q11 /2018-06-Beijing/WD11-24-FiberHome-CT-25G_50G_Flex025n_Mux'-and_OH_Structures.docx

- *OTN Over Packet Fabric Protocol (OFP) Implementation Agreement,* November 2011
- *Recommendation for Block Cipher Modes of Operation: Galois/Counter Mode (GCM) and GMAC,* November 2007